# EUROPÄISCHE PATENTANMELDUNG

(11) **EP 1 995 238 A2**
(43) Veröffentlichungstag der Anmeldung: **26.11.2008**
(21) Anmeldenummer: 08162452.0
(22) Anmeldetag: 05.07.2005
(51) Int. Cl.: C07D 209/82, A61K 31/403, A61P 15/00, A61P 35/00, A61P 43/00

(54) **Neue Tetrahydrocarbazolderivate mit verbesserter biologischer Wirkung und verbesserter Löslichkeit als Liganden für G-protein gekoppelte Rezeptoren (GPCR's)**

(30) Priorität: 14.07.2004 DE 102004033902; 14.07.2004 US 587969; 20.05.2005 US 683178
(62) Teilanmeldung aus: 05764033.6
(71) Anmelder: Æterna Zentaris GmbH, 60314 Frankfurt am Main (DE)
(72) Erfinder: Paulini, Klaus, 63477, Maintal (DE); Gerlach, Matthias, 63636, Brachttal (DE); Günther, Eckhard, 63477, Maintal (DE); Polymeropoulos, Emmanuel, 60325, Frankfurt am Main (DE); Baasner, Silke, 61137, Schöneck (DE); Schmidt, Peter, 61137, Schöneck (DE); Kühne, Ronald, 12683, Berlin (DE); Söderhäll, Arvid, 16341, Spanga (SE)

(57) **Zusammenfassung**

Die vorliegende Erfindung stellt neue, in ihren Eigenschaften verbesserte Tetrahydrocarbazol-Derivate bereit, die als Inhibitoren für GPCR's eingesetzt werden können. Damit ergibt sich die Möglichkeit, mit den neuen Verbindungen Krankheitszustände zu behandeln, die in ihrer Ausprägung von der pathobiochemischen Wirkung von GPCR's abhängen. Insbesondere wirken die erfindungsgemäßen Verbindungen über eine antagonistische Hemmung des LHRH-Rezeptors. Die Erfindung stellt ferner Arzneimittel bereit, die eine oder mehrere der neuen Verbindungen als Wirkstoff enthalten. Die Arzneimittel sind insbesondere geeignet, in einer oralen Darreichungsform am Säugetier oder Menschen eingesetzt zu werden.

## Beschreibung

Die vorliegende Erfindung betrifft neue Tetrahydrocarbazolderivate mit verbesserter biologischer Wirkung, verbesserter oraler Bioverfügbarkeit und verbesserter metabolischer Stabilität, als Liganden von G-Protein gekoppelten Rezeptoren (GPCR's), insbesondere als Liganden des Rezeptors für Luteinisierendes Hormon Releasinghormon (LHRH-Rezeptor), deren Herstellung, sowie deren Verwendung in pharmazeutischen Zusammensetzungen zur Behandlung von durch G-Protein gekoppelte Rezeptoren vermittelten Krankheitszuständen in einem Säugetier und insbesondere in einem Menschen.

### Hintergrund der Erfindung

Der Inhalt aller in dieser Anmeldung zitierten Publikationen oder vergleichbarer Quellen, welche herangezogen werden, um den Hintergrund der Erfindung zu erläutern, werden zum Zwecke der Offenbarung auch zum Gegenstand der vorliegenden Anmeldung gemacht.

G-Protein gekoppelte Rezeptoren stellen eine Superfamilie von zellmembranassoziierten Rezeptoren dar, die eine wichtige Rolle bei zahlreichen biochemischen und pathobiochemischen Vorgängen in Säugetieren und insbesondere im Menschen spielen. Alle GPCR's bestehen aus sieben hydrophoben, transmembranen alphahelicalen Domänen, die durch drei intra- und drei extrazelluläre Loops miteinander verbunden sind und über einen extrazellulären Amino- und einen intrazellulären Carboxy-Terminus verfügen. An ihrer zellulären Signaltransduktion sind ein oder mehrere heterotrimere G-Proteine beteiligt. Vielfältige physiologische Reize, wie Lichtempfindlichkeit, Geschmack und Geruch, aber auch grundlegende Prozesse wie Metabolismus, Reproduktion und Entwicklung werden über sie vermittelt und geregelt. Es existieren GPCR's für exogene und endogene Liganden. Peptidhormone, biogene Amine, Aminosäuren, Nucleotide, Lipide, Ca²⁺ aber auch Photonen wurden unter anderem als Liganden identifiziert; dabei kann ein Ligand verschiedene Rezeptoren aktivieren.

Nach einer jüngeren Untersuchung wurden im humanen Genom 367 Sequenzen für G-Protein gekoppelte Rezeptoren (GPCR's) mit endogenen Liganden identifiziert, D. K. Vassilatis et al., PNAS 100(8), 4903-4908 (2003). Davon zählen 284 zur Klasse A, 50 zur Klasse B, 17 zur Klasse C und 11 zur Klasse F/S. Zur Klasse A gehören beispielsweise der Bombesin-, der Dopamin- und der LHRH-Rezeptor und zur Klasse B der VIP- und der Calcitonin-Rezeptor. Für zahlreiche GPCR's sind die natürlichen Liganden noch nicht bekannt.

Aufgrund ihrer Funktion sind GPCR's als Angriffspunkte für Medikamente zur Therapie und Prävention einer großen Zahl von Krankheitszuständen geeignet. Es wird spekuliert, dass etwa 50% der gegenwärtig bekannten Wirkstofftargets GPCR's sind [Y. Fang et al., DDT 8(16), 755-761 (2003)]. So spielen GPCR's eine wichtige Rolle bei pathologischen Prozessen wie z.B. Schmerz (Opioid Rezeptor), Asthma (β₂-Adrenoceptor), Migräne (Serotonin 5-HT1B/1D-Rezeptor), Krebs (LHRH-Rezeptor), Herzkreislauferkrankungen (Angiotensin Rezeptor), metabolischen Störungen (GHS Rezeptor) oder Depressionen (Serotonin 5-HT₁ₐ Rezeptor), K. L. Pierce et al., Nat. Rev. Mol. Cell Biol. 3, 639-650 (2002).

Allgemeine Informationen über die GPCR's findet man unter http://www.gpcr.org.

Die vorliegende Erfindung beschreibt neue, in ihren Eigenschaften verbesserte Liganden für GPCR's im Allgemeinen, wobei die durch die Erfindung bereitgestellten Verbindungen insbesondere als Antagonisten des LHRH-Rezeptors wirken.

Der natürliche Ligand dieses Rezeptors, das Peptidhormon LHRH, wird in Zellen des Hypothalamus synthetisiert und pulsatil von den hypothalamischen Neuronen in den Kapillarplexus der Ementia mediana abgegeben. In der Adenohypophyse bindet das LHRH an die LHRH-Rezeptoren der gonadotropen Zellen und stimuliert bestimmte trimere G-Proteine, welche eine verzweigte Signaltransduktionskaskade anstoßen. Initial erfolgt eine Aktivierung von Phospholipase C, A₂ und/oder D. Dies führt zu einer gesteigerten Bereitstellung der Second Messenger Diacylglycerol und IP₃, gefolgt von einer Ca²⁺ -Mobilisierung aus intrazellulären Pools sowie einer Aktivierung verschiedener nachgeordneter Proteinkinasen. Letztlich kommt es zu einer Anregung der Produktion und zeitlich definierten, pulsativen Freisetzung der Gonadotropine FSH und LH. Beide Hormone werden über den Kreislauf zu den Zielorganen, Hoden bzw. Eierstöcken, transportiert. Dort stimulieren sie die Produktion und Freisetzung der entsprechenden Sexualhormone. Gegenläufig gibt es einen komplexen Rückkopplungsmechanisums, über den die Konzentration der gebildeten Sexualhormone wiederum die Freisetzung von LH und FSH reguliert.

Im männlichen Organismus bindet LH an Membranrezeptoren der Leydig-Zellen und stimuliert die Testosteron-Biosynthese. Das FSH wirkt über spezifische Rezeptoren an den Sertoli-Zellen und unterstützt die Produktion der Spermatozoen. Im weiblichen Organismus bindet LH an die LH-Rezeptoren der Thecazellen und aktiviert die Bildung von Androgen-synthetisierenden Enzymen. FSH stimuliert über die FSH-Rezeptoren an den Granulosazellen bestimmter Follikelstadien deren Proliferation. Die gebildeten Androgene werden in den benachbarten Granulosazellen in die Östrogene Östron und Östradiol umgewandelt.

Eine Reihe von Erkrankungen, die sich durch gutartige oder bösartige Wucherungen von Geweben auszeichnen, sind von der Stimulation durch Sexualhormone, wie Testosteron oder Östradiol abhängig. Typische Erkrankungen dieser Art beim Mann sind Prostatakrebs und benigne Prostatahyperplasie (BPH) sowie bei der Frau Endometriose, uterine Fibroide bzw. Uterus-Myome, Pubertas praecox, Hirsutismus und Polyzystisches Ovarielles Syndrom sowie Brustkrebs, Gebärmutterkrebs, Endometriumkrebs, Gebärmutterhalskrebs und Eierstockkrebs.

Seit seiner Entdeckung 1971 durch Schally et al. Science 173, 1036-1038 (1971) wurden mehr als 3000 synthetische Analoga zum natürlichen LHRH synthetisiert und getestet. Peptidische Agonisten wie Triptorelin und Leuprolide sind seit vielen Jahren erfolgreich in die Therapie von gynäkologischen Erkrankungen und Krebserkrankungen eingeführt. Agonisten haben allerdings generell den Nachteil, dass sie in der ersten Phase der Anwendung die LHRH-Rezeptoren stimulieren und so über eine initiale Erhöhung der Sexualhormonspiegel zu Nebenwirkungen führen. Erst nach Downregulierung des LHRH-Rezeptors infolge dieser Überstimulierung können die Superagonisten ihre Wirkung entfalten. Diese führt zu einer vollständigen Absenkung der Sexualhormonspiegel und damit zur pharmakologischen Kastration mit allen klinischen Symptomen. Dieser Nachteil ist verbunden mit der fehlenden Möglichkeit, den Spiegel an Sexualhormonen gezielt über die Dosierung einzustellen. Krankheiten, die keine totale Absenkung der Sexualhormonspiegel auf Kastrationsniveau erfordern, wie z.B. gutartige Wucherungen von Geweben, sind so für den Patienten mit einem Agonisten nicht optimal zu therapieren.

Dies führte zur Entwicklung der peptidischen LHRH-Rezeptor-Antagonisten, von denen z.B. Cetrorelix (Cetrotide^{®}) erfolgreich für die kontrollierte ovarielle Stimulation im Rahmen der Behandlung der weiblichen Unfruchtbarkeit eingeführt wurde. Die Antagonisten hemmen den LHRH-Rezeptor sofort und dosisabhängig, und führen somit zu einer unmittelbaren Absenkung der Plasmaspiegel von Testosteron bzw. Östradiol und Progesteron. Die peptidischen Antagonisten sind allerdings etwas weniger potent als die Agonisten und müssen somit höher dosiert werden.

Einen Überblick über die klinischen Anwendungen und das Potential von LHRH-Agonisten und Antagonisten geben R. P. Millar et al. in British. Med Bull. 56, 761-772 (2000) und R. E. Felberbaum et al., Mol. Cell. Endocrinology 166, 9-14 (2000), sowie F. Haviv et al. in Integration of Pharmaceutical Discovery and Development: Case Studies, Chapter 7, ed. Borchardt et al., Plenum Press, New York (1998). Neben der Behandlung von malignen und benignen Tumorerkrankungen kommen auch die kontrollierte ovarielle Stimulation im Rahmen einer künstlichen Befruchtung (In vitro Fertilisation), Fertilitätskontrolle (Kontrazeption) sowie Schutz vor unerwünschten Nebenwirkungen einer Radio- oder Chemotherapie, die Behandlung von HIV Infektionen (AIDS) und von neurologischen oder neurodegenerativen Erkrankungen wie der Alzheimer-Krankheit als mögliche Anwendungen in Frage. Spezifische LHRH-Rezeptoren wurden nicht nur bei Hypophysenzellen, sondern auch an Zellen in verschiedenen Tumoren z.B. der Brust und der Eierstöcke, gefunden. Diese Rezeptoren könnten einen direkten antiproliferativen Effekt von LHRH-Rezeptor-Antagonisten auf den Tumor vermitteln.

Bei den peptidischen LHRH-Rezeptor-Agonisten und -Antagonisten handelt es sich zumeist um Decapeptide, die für eine orale Gabe nicht ausreichend bioverfügbar sind. Sie werden typischerweise als Injektionslösungen oder als Depotformulierung subcutan oder intramuskulär gegeben. Bei der Anwendung ist dies mit Unannehmlichkeiten für den Patienten verbunden, und die Compliance leidet. Darüber hinaus ist die Synthese der Decapeptide aufwändig und teuer.

Es ist deshalb sinnvoll, nach nicht-peptidischen LHRH-Rezeptor-Antagonisten zu suchen, die neben hoher Wirksamkeit eine verbesserte metabolische Stabilität besitzen und oral verabreicht werden können.

### Stand der Technik

Im Vergleich zu den peptidischen LHRH-Rezeptor-Agonisten bzw. -Antagonisten ist noch keine nicht-peptidische Verbindung für eine der möglichen Indikationen zugelassen und in der klinischen Anwendung. Der gegenwärtige Entwicklungsstand auf dem Gebiet der LHRH-Rezeptor-Agonisten und -Antagonisten wird in den Übersichten von Y.-F. Zhu et al., Expert Opin. Therap. Patents 14(2), 187-199 (2004), Y.-F. Zhu et al., Ann. Rep. Med Chem. (39), 99-110 (2004), F.C. Tucci et al., Curr. Opin. Drug Discovery & Development 7(6), 832-847, (2004), R. E. Armer, Curr. Med Chem. 11, 3017-3028 (2004) und M. V. Chengalvala et al., Curr. Med. Chem.-Anti-CancerAgents, 3, 399-410 (2003) dargestellt. In der erstgenannten Publikation findet sich eine umfassende Auflistung der offengelegten Patentschriften, die die Synthese und Anwendung niedermolekularer LHRH-Rezeptor-Antagonisten beschreiben.

Zu den ersten Beispielen von nicht-peptidischen LHRH-Rezeptor-Antagonisten gehört die 4-Oxothieno[2,3-b]pyridin-Struktur, welche von N. Cho et al. in J. Med. Chem. 41, 4190-4195 (1998) beschrieben wurde. Diese Verbindungen, wie z.B. T-98475, haben zwar eine hohe Rezeptoraffinität, sind aber sehr schlecht wasserlöslich und haben eine geringe Bioverfügbarkeit. Basierend auf dieser Leitstruktur wurden zahlreiche Weiterentwicklungen durchgeführt, beispielhaft seien die Veröffentlichungen der internationalen Anmeldungen WO 95/28405, WO 96/24597, WO 97/14697 und WO 97/41126 genannt. Die Synthese von Thieno[2,3-d]pyrimidin-2,4-dionen als oral verfügbare LHRH-Rezeptor-Antagonisten wird von S. Sasaki et al., in J. Med. Chem. 46, 113-124 (2003) beschrieben.

Neue 1-Arylmethyl-5-aryl-6-methyluracile werden von Z. Guo et al., in J. Med. Chem. 47, 1259-1271 (2004) beschrieben. Die Herstellung von N-[(hetero)arylmethyl]benzene-sulfonamiden als potente nicht-peptidische LHRH-Rezeptor-Antagonisten wird in WO 03/078398 offenbart. In der Patentanmeldung WO 02/11732 werden tricyclische Pyrrolidine als LHRH-Rezeptor-Antagonisten beschrieben. Substituierte Pyridin-4-one als LHRH-Rezeptor-Antagonisten werden in WO 03/13528 und substituierte 1,3,5-Triazine-2,4,6-trione in WO 03/11839 offengelegt.

Die Synthesen und biologischen Aktivitäten von Erythromycin A-Derivaten mit LHRH-Rezeptor- antagonistischer Aktivität wird von J. T. Randolph et al., in J. Med Chem. 47(5), 1085-1097 (2004) beschrieben. Ausgewählte Derivate zeigen eine orale Wirksamkeit auf den LH-Spiegel am Modell der kastrierten Ratte.

Chinolin-Derivate als nicht-peptidische LHRH-Antagonisten werden beispielsweise in WO 97/14682 offenbart. Substituierte 2-Arylindole werden u. a. in WO 97/21435, WO 97/21703, WO 98/55116, WO 98/55470, WO 98/55479, WO 99/21553, WO 00/04013 als LHRH-Rezeptor-Antagonisten beschrieben. Entsprechend substituierte Aza-2-arylindole werden u. a. in WO 99/51231, WO 99/51596, WO 00/53178 und WO 00/53602 als LHRH-Rezeptor-Antagonisten beansprucht. Vorteilhafte biologische oder biophysikalische Daten zu diesen Verbindungen werden nicht offenbart.

Im Patent EP 0 679 642 B1 werden kondensierte heterocyclische Verbindungen als LHRH-Rezeptor-Antagonisten beschrieben. Der Tetrahydrocarbazolgrundkörper ist jedoch nicht Gegenstand der darin beschriebenen Erfindung.

1,2,3,4-Tetrahydrocarbazolcarbonsäuren werden in der Patentschrift EP 0 239 306 B1 als Prostaglandin-Antagonisten beschrieben. Eine LHRH-Rezeptor-antagonistische Wirkung ist weder beschrieben noch nahegelegt. In der US-Patentschrift 3,970,757 sind Tetrahydrocarbazolderivate als "Gastric Anti Secretory Agents" offengelegt. Eine LHRH-Rezeptor-antagonistische Wirkung für diesen Strukturtyp ist jedoch weder beschrieben noch nahegelegt. In EP 603 432 B1 beziehungsweise US 5,708,187 werden Tetrahydrocarbazolderivate als 5-HT1 Agonisten u.a. zur Behandlung von Migräne beschrieben. Eine LHRH-Rezeptor-antagonistische Wirkung ist aber weder beschrieben noch nahegelegt. In WO 2005/033099 A2 werden Tetrahydrocarbazolderivate als Dipeptidyl Peptidase IV Inhibitoren beschrieben. Eine LHRH-Rezeptor-antagonistische Wirkung ist aber weder beschrieben noch nahegelegt. Es gibt keinen Hinweis auf LHRH-Rezeptor-antagonistische Wirkung, und die offenbarten Strukturen unterscheiden sich von den Verbindungen der vorliegenden Erfindung. D. J. Davies et al. beschreiben in J. Med. Chem. 41, 451- 467 (1998) Tetrahydrocarbazol-Derivate mit Melatoninagonistischer bzw. -antagonistischer Wirkung. Eine LHRH-Rezeptor-antagonistische Wirkung ist aber weder beschrieben noch nahegelegt. Tetrahydrocabazolderivate werden durch S.J. Shuttleworth et al. in Bioorg. Med Chem. Lett. 14, 3037-3042 (2004) als partielle Agonisten des Neuromedin B Rezeptors beschrieben. Eine LHRH-Rezeptor-antagonistische Wirkung ist aber weder beschrieben noch nahegelegt. R. Millet et al. beschreiben in Letters in Peptide Science 6, 221-233 (1999) Tetrahydrocarbazolderivate als NK₁/NK₂ Liganden. Die offenbarten Strukturen unterscheiden sich von den Verbindungen der vorliegenden Erfindung. Eine LHRH-Rezeptor-antagonistische Wirkung ist darüber hinaus weder beschrieben noch nahegelegt. Die Festphasensynthese von 3-Amino-3'-carboxytetrahydrocarbazolen wird bei Koppitz et al., THL 46(6), 911-914 (2005). Eine LHRH-Rezeptor-antagonistische Wirkung ist weder beschrieben noch nahegelegt.

Tetrahydrocarbazol-Derivate als peptidomimetische LHRH-Rezeptor-Antagonisten mit guter Rezeptoraffinität werden beispielsweise in WO 03/051837 (DE 101 64 564 A1) offenbart. Die physikochemischen und metabolischen Eigenschaften dieser Verbindungen machen sie aber nicht in optimaler Weise für eine orale Darreichungsform geeignet.

Eine Reihe von Publikationen geben Überblick über den Entwicklungsstand von Neurokinin Antagonisten. G. Giardina et al., IDrugs 6(8), 758-772 (2003), geben einen Überblick über die aktuelle Patentliteratur. V. Leroy et al., Expert Opinion on Investigational Drugs 9(4), 735-746 (2000), und C. Swain et al., Annual Reports in Medicinal Chemistry 34, 51-60 (1999) beschreiben den Entwicklungsstand bezüglich Neurokinin Rezeptor Antagonisten während z.Bsp. R.M. Navari et al., Cancer Investigation 22(4) 569-576 (2004), Ergebnisse klinischer Studien darlegt, bei denen NK1 Rezeptor Antagonisten gegen chemotherapieinduzierte Emesis eingesetzt wurden. R.G. Hill et al. beschreiben in Pain, 523-530 (2003) Neurokinin Rezeptor Antagonisten als potentielle Schmerzmittel, während A. von Sprecher et al., in IDrugs 1(1), 73-91 (1998), Neurokinin Rezeptor Antagonisten als potentielle Wirkstoffe für die Therapie von Entzündungen und rheumatoider Arthritis beschreiben. R. Millet et al. beschreiben in Letters in Peptide Science 6, 221-233 (1999) Tetrahydrocarbazolderivate als NK₁/NK₂ Liganden. Die offenbarten Strukturen unterscheiden sich von den Verbindungen der vorliegenden Erfindung.

### Aufgabe der Erfindung

Die vorliegende Erfindung hat die Aufgabe, neue Verbindungen mit verbesserter oraler Bioverfügbarkeit und verbesserter metabolischer Stabilität bereitzustellen, die zur Behandlung von durch GPCR's vermittelten Krankheitszuständen an Säugern und insbesondere am Menschen eingesetzt werden können. Vorzugsweise sollen die neuen Verbindungen über eine antagonistische Hemmung des LHRH-Rezeptors ihre biologische Wirkung entfalten. Die neuen Verbindungen sollen geeignet sein, in einer für die Anwendung akzeptablen Dosierung und in einer oralen Formulierung dosisabhängig den gewünschten Effekt zu erzielen. Dazu ist es erforderlich, dass die neuen Verbindungen als pharmakologisch aktive Wirkstoffe in einem Arzneimittel am Säugetier oder Menschen angewendet werden können.

Die erfinderische Aufgabe wird in überraschender Weise durch die Bereitstellung der neuen, verbesserten Tetrahydrocarbazol-Derivate gemäß nachstehender allgemeiner Formel (1) gelöst.

### Detaillierte Beschreibung der Erfindung

Ein erster Aspekt der vorliegenden Erfindung betrifft neue Tetrahydrocarbazol-Verbindungen der allgemeinen Formel (I): 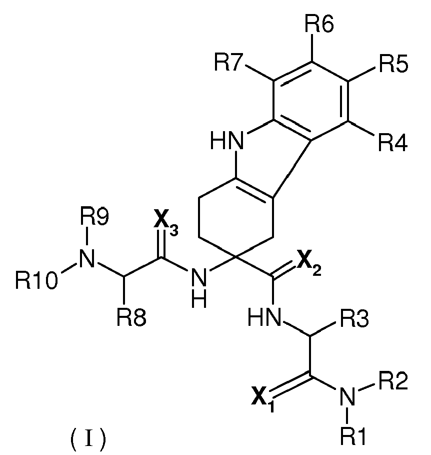 worin:
X₁ O bedeutet,
- X₂: und X₃ unabhängig voneinander O oder geminal verknüpftes H₂ bedeuten,
- R1 und R2: unabhängig voneinander ausgewählt sind aus der Gruppe bestehend aus -H, Aryl-, Alkyl- und Arylalkyl-Resten, welche gegebenenfalls in der Alkyl- und/oder Aryl-Gruppe mit bis zu 3 Substituenten, unabhängig ausgewählt aus der Gruppe bestehend aus -Hal, -CN, und -O-alkyl, substituiert sind, wobei R1 und R2 insbesondere jeweils ein Wasserstoffatom bedeuten,
- R3 und R8: einen Alkyl-, Arylalkyl- oder Heteroarylalkyl-Rest bedeutet, welche gegebenenfalls mit bis zu 3 Substituenten, unabhängig ausgewählt aus der Gruppe bestehend aus -Hal, -CN, -CO-O-R12, -CO-NR12R12', -OH, -O-R13, -O-CO-R13, -O-SO₂-OR12, -O-SO₂-R12, -SO₂-OR12, -SO-R12, -O-PO(OR12)(OR12'), -O-PO(NR12R12')₂, -O-CO-O-R13, -O-CO-NR12R12', -O-CS-NR12R12', -S-R12, -NR12R12', -NH-CO-R13, -NH-SO₂-R12, -NH-CO-O-R13, -NH-CO-NHR12, -NH-C(NH)-NH₂ substituiert sind,
- R4, R5, R6 und R7: unabhängig voneinander ausgewählt sind aus der Gruppe bestehend aus H, -Hal, -CN, -CONH₂, -COOH, -CF₃, -O-Alkyl, -OCF₃, -NO₂, und Alkyl-, Arylalkyl-, und Heteroarylalkyl-Resten;
- R9: ein Wasserstoffatom, einen Alkyl-, einen Aryl-, einen Heteroaryl-, einen Arylalkyl- oder einen Heteroarylalkyl-Rest, vorzugsweise ein Wasserstoffatom, bedeutet;
- R10: den Rest -R11, -CO-R11, -CO-OR11, -CO-NHR11, -C(NH)-NHR11, -SO₂-R11, oder -SO₂-NHR11 bedeutet;
- R11: ein Alkyl-, ein Aryl-, ein Heteroaryl-, ein Arylalkyl- oder ein Heteroarylalkyl-Rest bedeutet, welche gegebenenfalls mit einem oder mehreren Substituenten, unabhängig ausgewählt aus der Gruppe bestehend aus -Hal, -CN, -Alkyl, -CF₃, -OCF₃, -OH, -O-Alkyl, und -O-(CH₂CH₂-O)ₙ-CH₃, substituiert sind, wobei der Arylalkyl- oder Heteroarylalkyl-Rest in der Aryl- bzw. Heteroaryl-Gruppe mit einem oder mehreren Substituenten, vorzugsweise mit einem, zwei oder drei Substituenten, unabhängig ausgewählt aus der Gruppe bestehend aus Hal, -CN, -Alkyl, -CF₃, -OCF₃, -OH, -O-Alkyl, und -O-(CH₂CH₂-O)ₙ-CH₃ substituiert ist;
- R12 und R12': unabhängig voneinander H, oder einen Alkyl-, Arylalkyl-, Aryl-, Heteroarylalkyl-, oder Heteroaryl-Rest, vorzugsweise Wasserstoffe bedeuten,
- R13: ausgewählt ist aus einem Alkyl-, Arylalkyl-, Aryl-, Heteroarylalkyl-, und Heteroaryl-Rest, oder die Gruppe -(CH₂CH₂-O)ₙ-CH₃ bedeutet; und
n für eine ganze Zahl von 1 bis 10, vorzugsweise 1 bis 6, steht.

Die zur Erläuterung der Verbindungen der allgemeinen Formel (I) angegebenen Begriffe haben grundsätzlich, sofern in der Beschreibung oder in den Ansprüchen nichts anderes angegeben ist, folgende Bedeutungen:

Der Ausdruck "substituiert" bedeutet, daß der entsprechende Rest oder Gruppe einen oder mehrere Substitueten trägt. Sofern ein Rest mehrere Substituenten trägt und eine Auswahl verschiedener Subsituenten vorgegeben ist, werden die Substituenten unabhängig voneinander ausgewählt und müssen nicht identisch sein. Der Ausdruck "unsubstituiert" bedeutet, daß die entsprechende Gruppe keine Substituenten trägt. Der Ausdruck "gegebenenfalls substituiert" bedeutet, daß die entsprechende Gruppe entweder unsubstituiert oder durch einen oder mehrere Substituenten substituiert ist. Der Ausdruck "mit bis zu 3 Substituenten substituiert" bedeutet, dass der entsprechende Rest oder Gruppe entweder durch einen oder durch zwei oder drei Substituenten substituiert ist.

Der Ausdruck "Halogenatom" oder "Halogensubstituent" (Hal-) bezeichnet ein oder gegebenenfalls mehrere Fluor-, (F, Fluoro-), Brom (Br, Bromo-), Chlor- (Cl, Chloro), oder Jod (J, lodo-) Atome. Die Bezeichnungen "Dihalogen", "Trihalogen" und "Perhalogen" bezeichnen zwei, drei beziehungsweise vier Substituenten, wobei jeder Substituent unabhängig aus der Gruppe bestehend aus Fluor, Chlor, Brom und Jod ausgewählt sein kann. Bevorzugt bedeutet "Halogen" ein Fluor- oder Chlor-Atom.

Der Ausdruck "Alkyl" umfasst im Sinne dieser Erfindung acyclische gesättigte oder partiell ungesättigte Kohlenwasserstoffe mit C1-C12 Kohlenstoffatomen, die geradkettig oder verzweigt sein können. Vorzugsweise steht der Ausdruck "Alkyl" für Alkyl-Ketten aus 1 bis 8, besonders bevorzugt 1 bis 6 Kohlenstoffatomen. Beispiele für geeignete Alkyl-Reste sind Methyl, Ethyl, n-Propyl, iso-Propyl, n-Butyl, sec.-Butyl, tert-Butyl, n-Pentyl, tert-Pentyl, 2- oder 3-Methylpentyl, n-Hexyl, n-Heptyl, n-Octyl, n-Nonyl, n-Decyl, n-Undecyl, n-Dodecyl, Propenyl, Butenyl, Pentenyl, Hexenyl und Octadienyl. Ebenso steht der Ausdruck "Alkyl" für einen gesättigten oder partiell ungesättigten Cycloalkyl-Rest, bevorzugt aus der Gruppe Cyclo(C3-C8)alkyl. Beispiele für geeignete Cycloalkylreste sind Cyclopropyl, Cyclobutyl, Cyclopentyl, Cyclohexyl, Cycloheptyl, Cyclooctyl, Cyclohexenyl, Cyclopentenyl, Cyclooctadienyl und andere. Außerdem umfasst der Begriff "Alkyl" Cycloalkyl-Alkyl Gruppen, wobei der Cyclo(C3-C8)alkyl-(C1-C4)alkyl-Rest bevorzugt ist. Beispiele hierfür sind Cyclopropylmethyl, Cyclohexylmethyl, Cyclopentylethyl, Cyclohexenylethyl. So umfasst der Ausdruck C₁-C₄-Alkyl zumindest folgende Gruppen: Methyl, Ethyl, n-Propyl, iso-Propyl, Propenyl, Cyclopropyl, n-Butyl, sec.-Butyl, tert-Butyl, Cyclobutyl, Cyclopropylmethyl, und Butenyl. Besonders bevorzugt als C₁-C₄-Alkyl sind iso-Propyl, sec.-Butyl, sowie Cyclopropylmethyl.

Derartige, Alkyl-Rest können unsubstituiert oder gegebenenfalls auch einfach oder mehrfach substituiert vorliegen, wobei die Substituenten gleich oder verschieden und in jeder oder mehreren beliebigen und möglichen Position des Alkyls gebunden sein können. Im Falle eines ein- oder mehrfach Halogen-substituierten Alkylrests gilt, dass die Substitution mit Fluor- und/oder Chloratomen bevorzugt ist. Beispiele für derartige Reste sind Fluormethyl, Trifluormethyl und Pentafluorethyl.

"Aryl" bezeichnet aromatische Kohlenwasserstoff-Systeme mit 3 bis 14, vorzugsweise 5 bis 14 Kohlenstoffatomen, die auch mit weiteren gesättigten, (partiell) ungesättigten oder aromatischen Ringsystemen kondensiert sein können. Beispiele für "Aryl" sind u.a. Phenyle, Naphthyle und Anthracenyle, aber auch Indanyle, Indenyle, oder 1,2,3,4-Tetrahydro-naphthyle; besonders bevorzugt im Rahmen der vorliegenden Erfindung ist Phenyl. Derartige Aryl-Rest können unsubstituiert oder gegebenenfalls auch einfach oder mehrfach substituiert vorliegen, wobei die Substituenten gleich oder verschieden und in jeder oder mehreren beliebigen und möglichen Position des Aryls gebunden sein können.

"Heteroaryl" bezeichnet einen 5-, 6- oder 7-gliedrigen cyclischen aromatischen Rest, der mindestens 1, ggf. auch 2, 3, 4 oder 5 Heteroatome, bevorzugt Stickstoff, Sauerstoff und/oder Schwefel enthält, wobei die Heteroatome gleich oder verschieden sind. Die Zahl der N Atome liegt bevorzugt zwischen 0 und 3, und die der Sauerstoff- und Schwefelatome zwischen 0 und 1. Ferner umfaßt der Begriff "Heteroaryl" auch Systeme, in denen der Heterocyclus Teil eines bi- oder polyzyklischen Systems ist, wobei die Bindung des Heteroaryl-Restes an die Verbindungen der allgemeinen Formel (I) über jedes beliebige und mögliche Ringglied des Heteroaryl-Restes erfolgen kann. Beispiele für "Heteroaryl" sind u.a. Pyrrolyl, Thienyl, Furyl, Imidazolyl, Thiazolyl, Isothiazolyl, Oxazolyl, Isoxazolyl, Pyrazolyl, Pyridinyl, Pyrimidinyl, Pyrazinyl, Indolyl, Quinolinyl, und Isoquinolinyl. Derartige Heteroaryl-Reste können unsubstituiert oder gegebenenfalls ein- oder mehrfach substituiert sein, wobei die Substituenten gleich oder verschieden und in jeder oder mehreren beliebigen und möglichen Position des Heteroaryls gebunden sein können.

"Arylalkyl" oder "Heteroarylalkyl" bezeichnen Reste, in denen der Aryl- bzw. Heteroaryl-Rest über eine C₁-C₈-Alkyl-Gruppe an die Verbindung der allgemeinen Formel (I) gebunden ist, wobei die Alkyl-, Aryl- und Heteroaryl-Gruppen die vorstehend definierten Bedeutungen aufweisen. Bevorzugte "Arylalkyl" Gruppen sind Phenyl-C₁-C₄-alkyl-Reste, vorzugsweise Benzyl- oder Phenylethyl-Reste.

Ein "Ringsystem" bezeichnet ein mono-oder polyzyklisches System aus 3 bis 14, vorzugsweise 5 oder 6 bis 14 Ringatomen, die ausschließlich Kohlenstoffatome sein können. Das Ringsystem kann aber auch 1, 2, 3, 4, oder 5 Heteroatome, insbesondere Stickstoff, Sauerstoff und/oder Schwefel enthalten. Das Ringsystem kann gesättigt, ein- oder mehrfach ungesättigt oder ganz oder teilweise aromatisch sein, wobei im Falle eines mindestens aus zwei Ringen bestehenden Ringsystems die Ringe kondensiert, spiro- oder anderweitig verbunden sein können.

Wie vorstehend im Zusammenhang mit der allgemeinen Formel (I) erwähnt, können die erfindungsgemäßen Verbindungen, da sie mindestens ein Asymmetriezentrum aufweisen, in Form ihrer Racemate, in Form der reinen Enantiomeren und/oder Diastereomeren oder in Form von Mischungen dieser Enantiomeren und/oder Diastereomeren vorliegen. Die Mischungen können in jedem beliebigen Mischungsverhältnis der Stereoisomere vorliegen.

Bevorzugt sind jene Verbindungen der allgemeinen Formel (I), die an dem durch -NH-CX₃- und -CX₂-NH- substituierten Kohlenstoffatom in R-Konfiguration vorliegen, d.h. die folgende allgemeine Formel (I-a) aufweisen:

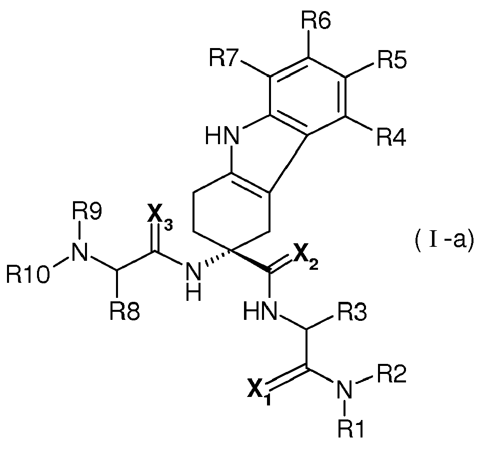

Besonders bevorzugt sind jene Verbindungen der allgemeinen Formel (I), die an dem durch -NH-CX₃- und -CX₂-NH- substituierten Kohlenstoffatom in R-Konfiguration, an dem durch -CX₃-NH-, -R8 und -NR9R10 substituierten Kohlenstoffatom in S-Konfiguration und an dem durch -NH-CX₂-, -R3 und -CX₁-NR1R2 substituierten Kohlenstoffatom ebenfalls in S-Konfiguration vorliegen, d.h. an diesen Stereozentren die natürlich vorkommende S-Konfiguration der entsprechenden Aminosäuren haben. Diese Verbindungen weisen die folgende allgemeine Formel (I-b) auf:

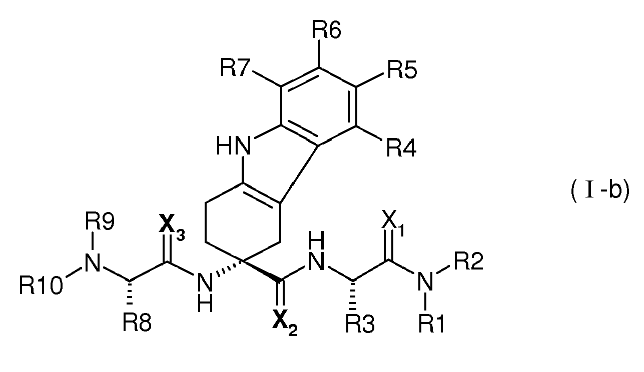

Sofern möglich, können die erfindungsgemäßen Verbindungen in Form der Tautomeren vorliegen.

So lassen sich beispielsweise die erfindungsgemäßen Verbindungen gemäß der allgemeinen Formel (I), welche ein oder mehrere Chiralitätszentren aufweisen und die als Racemate bzw. als Diastereomerengemische auftreten, nach an sich bekannten Methoden in ihre optischen reinen Isomeren, also Enantiomere oder Diastereomere auftrennen. Die Trennung der erfindungsgemäßen Verbindungen oder ihrer Bausteine (Aminosäuren) kann durch Säulentrennung an chiralen oder nicht-chiralen Phasen oder durch Umkristallisation, aus einem gegebenenfalls optisch aktiven Lösungsmittel oder unter Verwendung einer optisch aktiven Säure oder Base oder durch Derivatisierung mit einem optisch aktiven Reagenz, wie beispielsweise einem optisch aktiven Alkohol, und anschließender Abspaltung des Restes erfolgen.

Die erfindungsgemäßen Verbindungen der allgemeinen Formeln (I, la und Ib) können, falls sie eine ausreichend basische Gruppe, wie zum Beispiel ein sekundäres oder tertiäres Amin besitzen, mit anorganischen und organischen Säuren in Salze überführt werden. Vorzugsweise werden die pharmazeutisch annehmbaren Salze der erfindungsgemäßen Verbindungen gemäß der allgemeinen Formeln (I, Ia und Ib) mit Salzsäure, Bromwasserstoffsäure, Schwefelsäure, Phosphorsäure, Methansulfonsäure, p-Toluolsulfonsäure, Kohlensäure, Ameisensäure, Essigsäure, Sulfoessigsäure, Trifluoressigsäure, Oxalsäure, Malonsäure, Maleinsäure, Bernsteinsäure, Weinsäure, Traubensäure, Äpfelsäure, Embonsäure, Mandelsäure, Fumarsäure, Milchsäure, Citronensäure, Taurocholinsäure, Glutaminsäure oder Asparaginsäure gebildet. Bei den gebildeten Salzen handelt es sich u.a. um Hydrochloride, Hydrobromide, Sulfate, Phosphate, Methansulfonate, Tosylate, Carbonate, Hydrogencarbonate, Formiate, Acetate, Sulfoacetate, Triflate, Oxalate, Malonate, Maleate, Succinate, Tartrate, Malate, Embonate, Mandelate, Fumarate, Lactate, Citrate und Glutaminate. Die Stöchiometrie der gebildeten Salze der erfindungsgemäßen Verbindungen kann dabei ganzzahlige oder nicht ganzzahlige Vielfache von eins betragen.

Die erfindungsgemäßen Verbindungen der allgemeinen Formeln (I, Ia und Ib) können, falls sie eine ausreichend saure Gruppe, wie zum Beispiel die Carboxy-, Sulfonsäure-, Phosphorsäure- oder eine phenolische Gruppe enthalten, mit anorganischen und organischen Basen in ihre physiologisch verträglichen Salze überführt werden. Als anorganische Basen kommen beispielsweise Natriumhydroxid, Kaliumhydroxid, Calciumhydroxid, als organische Basen Ethanolamin, Diethanolamin, Triethanolamin, Cyclohexylamin, Dibenzylethylendiamin und Lysin in Betracht. Die Stöchiometrie der gebildeten Salze der erfindungsgemäßen Verbindungen kann dabei ganzzahlige oder nicht ganzzahlige Vielfache von eins betragen.

Ebenfalls können die erfindungsgemäßen Verbindungen der allgemeinen Formeln (I, Ia und Ib) in Form ihrer Solvate und insbesondere Hydrate vorliegen, die z. B. durch Kristallisation aus einem Lösungsmittel oder aus wässriger Lösung erhalten werden können. Es können sich dabei ein, zwei, drei oder beliebig viele Solvat- oder WasserMoleküle mit den erfindungsgemäßen Verbindungen zu Solvaten und Hydraten verbinden.

Es ist bekannt, dass chemische Substanzen Festkörper ausbilden, die in verschiedenen Ordnungszuständen vorliegen, die man als polymorphe Formen oder Modifikationen bezeichnet. Die verschiedenen Modifikationen einer polymorphen Substanz können sich in ihren physikalischen Eigenschaften stark unterscheiden. Die erfindungsgemäßen Verbindungen der allgemeinen Formeln (I, Ia und Ib) können in verschiedenen polymorphen Formen vorliegen, dabei können bestimmte Modifikationen metastabil sein. Alle diese polymorphen Formen der Verbindungen der allgemeinen Formeln (I, Ia und Ib) sind als zur Erfindung gehörig anzusehen.

Ebenfalls können die erfindungsgemäßen Verbindungen der allgemeinen Formeln (I, Ia und Ib) in Form beliebiger Prodrugs wie beispielsweise Ester, Karbonate oder Phosphate vorkommen, bei denen die tatsächlich biologisch aktive Form erst durch Verstoffwechselung freigesetzt wird.

Es ist bekannt, dass chemische Substanzen im Körper zu Metaboliten umgewandelt werden die gegebenenfalls ebenfalls die erwünschte biologische Wirkung - unter Umständen sogar in stärker ausgeprägter Form - hervorrufen können.

Entsprechende Metaboliten der Verbindungen der allgemeinen Formeln (I, la und Ib) sind als zur Erfindung gehörig anzusehen.

Weiterhin sei an dieser Stelle bemerkt, dass die Bezeichnung "Rezeptorligand" oder "Ligand" für die Zwecke der vorliegenden Erfindung jede Verbindung bezeichnen soll, die in irgendeiner Weise an einen Rezeptor bindet (in der vorliegenden Erfindung ist der Rezeptor ein GPCR-Rezeptor, vorzugsweise ein LHRH-Rezeptor) und entweder eine Aktivierung, Inhibierung und/oder sonstige erdenkliche Wirkung bei diesem Rezeptor auslöst. Der Ausdruck "Ligand" umfasst somit Agonisten, Antagonisten, Partial-Agonisten/Antagonisten und sonstige Liganden, die beim Rezeptor eine Wirkung hervorrufen, die der Wirkung von Agonisten, Antagonisten oder Partial-Agonisten/Antagonisten ähnlich ist. Vorzugsweise sind die erfindungsgemäßen Verbindungen der allgemeinen Formeln (I, Ia und Ib) Antagonisten des LHRH-Rezeptors (GnRH Rezeptors).

Bevorzugte Verbindungen der Formeln (I, Ia und Ib) im Rahmen der vorliegenden Erfindung zeichnen sich dadurch aus, daß X₁ ein O-Atom, bedeutet, und R8 für einen Alkyl-, Arylalkyl- oder Heteroarylalkyl-Rest steht, wobei diese Reste gegebenenfalls mit bis zu 3 Substituenten, unabhängig ausgewählt aus der Gruppe bestehend aus -Hal, -CN, -CO-O-R12, -CO-NR12R12', -OH, -O-R13, -O-CO-R13, -O-SO₂-OR12, -O-SO₂-R12, -SO₂-OR12, -SO-R12, -O-PO(OR12)(OR12'), -O-PO(NR12R12')₂, -O-CO-O-R13, -O-CO-NR12R12', -O-CS-NR12R12', -S-R12, -NR12R12', -NH-CO-R13, -NH-SO₂-R12, -NH-CO-O-R13, -NH-CO-NHR12, -NH-C(NH)-NH₂ substituiert sind, wobei R12, R12' und R13 die oben angegebenen Bedeutungen haben.

Weiterhin bevorzugte Verbindungen der Formeln (I, Ia und Ib) im Rahmen der vorliegenden Erfindung zeichnen sich dadurch aus, daß
- R10: den Rest -R11, -CO-R11, -CO-OR11, -CO-NHR11, -C(NH)-NHR11, -SO₂-R11, oder -SO₂NHR11 bedeutet,
- R11: ein Arylalkyl- oder Heteroarylalkyl-Rest ist, der in der Aryl- bzw. Heteroaryl-Gruppe mit einem oder mehreren Substituenten, unabhängig ausgewählt aus der Gruppe bestehend aus Hal, -CN, -Alkyl, -CF₃, -OCF₃, -OH, -O-Alkyl, und -O-(CH₂CH₂-O)ₙ-CH₃ substituiert ist, und
- R8: einen Alkyl-, Arylalkyl- oder Heteroarylalkyl-Rest bedeutet, der gegebenenfalls mit bis zu 3 Substituenten, unabhängig ausgewählt aus der Gruppe bestehend aus -Hal, -CN, -CO-O-R12, -CO-NR12R12', -OH, -O-R13, -O-CO-R13, -O-SO₂-OR12, -SO₂-OR12, -O-SO₂-R12, -SO-R12, -O-PO(OR12)(OR12'), -O-PO(NR12R12')₂, -O-CO-O-R13, -O-CO-NR12R12', -O-CS-NR12R12', -S-R12, -N R12R12', -NH-CO-R13, -NH-SO₂-R12, -NH-CO-O-R13, -NH-CO-NHR12, -NH-C(NH)-NH₂ substituiert ist, wobei R12, R12' und R13 die oben angegebenen Bedeutungen haben.

In einer bevorzugten Variante der vorliegenden Erfindung sind mindestens einer, vorzugsweise zwei der Reste R4, R5, R6, und R7, vorzugsweise R5 und R7, keine Wasserstoffatome. Insbesondere sind die Reste R5 und R7 unabhängig voneinander ausgewählt aus der Gruppe bestehend aus -H, -Hal, -CN, -CF₃, -O-Alkyl und -OCF₃, und bedeuten vorzugsweise -H, -Hal oder -CF₃. Insbesondere sind Verbindungen bevorzugt, bei denen R4 und R6 jeweils ein Wasserstoffatom, R5 entweder -H oder - Hal, und R7, unabhängig von R5, entweder -Hal oder -CF₃ bedeuten.

Weiterhin bevorzugte Verbindungen der Formeln (I, Ia und Ib) im Rahmen der vorliegenden Erfindung zeichnen sich dadurch aus, daß X₂ und X₃ jeweils O bedeuten.

In einer bevorzugten Variante der vorliegenden Erfindung bedeutet R3 einen C₁-C₆-Alkyl-Rest, vorzugsweise einen C₁-C₄-Alkyl-Rest.

Bevorzugte Verbindungen im Rahmen der vorliegenden Erfindung zeichnen sich dadurch aus, dass R1, R2, R9 und auch R12 und R12', sofern vorhanden, jeweils ein Wasserstoffatom bedeuten.

Weiterhin bevorzugt sind Verbindungen der allgemeinen Formeln (I, la und Ib), bei denen R13 einen Phenyl-C₁-C₄-alkyl-Rest, oder die Gruppe -(CH₂CH₂-O)ₙ-CH₃ bedeutet.

In bevorzugten Verbindungen der Formeln (I, Ia und Ib) weist R10 die Bedeutung -CO-R11, -CO-OR11 oder-R11 auf, wobei R11 die oben angegebenen Bedeutungen hat.

Weiterhin gelten Verbindungen der allgemeinen Formeln (I, Ia und Ib) als bevorzugt, in denen R11 einen Phenyl-C₁-C₄-alkyl-Rest, vorzugsweise einen Benzyl- oder Phenylethyl-Rest bedeutet, der in der Phenyl-Gruppe gegebenenfalls mit einem bis drei, vorzugsweise einem oder zwei Substituenten, unabhängig ausgewählt aus der Gruppe bestehend aus -Hal, -C₁-C₄-Alkyl, -CF₃, -OCF₃, -OH, -O-C₁-C₄-Alkyl und -O-(CH₂CH₂-O)ₙ-CH₃ substituiert ist.

Im Rahmen der vorliegenden Erfindung sind insbesondere Verbindungen der allgemeinen Formeln (I, Ia und Ib) von Interesse, wobei
- X₁: O bedeutet,
- X₂ und X₃: jeweils O bedeuten,
- R1 und R2: jeweils ein Wasserstoffatom bedeuten,
- R3: einen C₁-C₆-Alkyl-Rest, vorzugsweise einen C₁-C₄-Alkyl-Rest bedeutet,
- R4 und R6: jeweils ein Wasserstoffatom bedeuten,
- R5: entweder ein Wasserstoffatom oder Hal bedeutet,
- R7: entweder Hal oder -CF₃ bedeutet,
- R9: ein Wasserstoffatom bedeutet,
- R10: den Rest -CO-R11 oder -CO-OR11 oder den Rest R11 bedeutet,
- R11: ein Phenyl-C₁-C₄-alkyl-Rest, vorzugsweise ein Benzyl- oder Phenylethyl-Rest ist, der in der Phenyl-Gruppe gegebenenfalls mit einem bis drei, vorzugsweise einem oder zwei Substituenten, unabhängig ausgewählt aus der Gruppe bestehend aus -Hal, -C₁-C₄-Alkyl, -CF₃, -OCF₃, -OH, -O-C₁-C₄-Alkyl und -O-(CH₂CH₂-O)ₙ-CH₃ substituiert ist, und
- R8: einen C₁-C₆-Alkyl-, vorzugsweise einen C₁-C₄-Alkyl-Rest bedeutet, und
- n: für eine ganze Zahl von 1 bis 6, vorzugsweise 1 bis 4, steht.

Insbesondere die folgenden Verbindungen der allgemeinen Formel (I), bzw. (I-a) oder (I-b) sind als besonders bevorzugt anzusehen:

Verbindungen, bei denen X₁ O bedeutet, R₃ einen C₁-C₄-Alkyl-Rest bedeutet, R4 und R6 jeweils ein Wasserstoffatom bedeuten, R₅ und R₇ jeweils Hal bedeuten, oder R₅ ein Wasserstoffatom und R7 die Gruppe -CF₃ bedeutet, R10 den Rest -CO-R11 oder-CO-OR11 oder den Rest R11 bedeutet, R11 ein Benzyl- oder Phenylethyl-Rest ist, der in der Phenyl-Gruppe mit einem oder zwei Hal-Atomen substituiert ist, und R8 einen C₁-C₄-alkyl, Benzyl- oder Phenylethyl-Rest bedeutet, wobei der Phenyl-Rest gegebenenfalls mit -OH substituiert ist.

Am meisten bevorzugt sind folgende Verbindungen gemäß der allgemeinen Formel (I):
{(S)-1-[(R)-3-((S)-1-Carbamoyl-2-methyl-propylcarbamoyl)-6,8-dichlor-2,3,4,9-tetrahydro-1H-carbazol-3-yl-carbamoyl]-2-methyl-propyl}-carbaminsäure-4-chlobenzylester (**1**),
{(S)-1-[(R)-3-((S)-1-Carbamoyl-2-methyl-propylcarbamoyl)-6,8-dichlor-2,3,4,9-tetrahydro-1H-carbazol-3-yl-carbamoyl]-2-methyl-butyl}-carbaminsäure-4-ch10r-benzylester (**2**),
{(S)-1-[(R)-3-((S)-Carbamoyl-2-methyl-butylcarbamoyl)-6,8-dichlor-2,3,4,9-tetrahydro-1H-carbazol-3-yl-carbamoyl]-2-methyl-butyl}-carbaminsäure-4-chlor-benzylester (**3**),
(R)-6,8-Dichlor-3-{(S)-2-[2-(2-fluorphenyl)-acetylamino]-3-methyl-pentanoylaminol-2,3,4,9-tetrahydro-1H-carbazol-3-carbonsäure-((S)-1-carbamoyl-2-methyl-butyl)-amid (**4**),
(R)-6,8-Dichlor-3-{(S)-2-[2-(3-fluorphenyl)-acetylamino]-3-methyl-pentanoylaminol-2,3,4,9-tetrahydro-1H-carbazol-3-carbonsäure-((S)-1-carbamoyl-2-methyl-butyl)-amid (**5**),
{(S)-1-[(R)-3-((S)-Carbamoyl-2-methyl-butylcarbamoyl)-6,8-dichlor-2,3,4,9-tetrahydro-1H-carbazo1-3-yl-carbamoyl]-2-methyl-butyl}-carbaminsäure-2-chlor-benzylester (**6**),
(R)-6,8-Dichlor-3-{(S)-2-[3-(4-fluorphenyl)-propionylamino]-3-methyl-pentanoylaminol-2,3,4,9-tetrahydro-1H-carbazol-3-carbonsäure-((S)-1-carbamoyl-2-methyl-propyl)-amid (**16**),
(S)-5-[(R)-3-((S)-1-Carbamoyl-2-methyl-propylcarbamoyl)-6,8-dichlor-2,3,4,9-tetrahydro-1H-carbazol-3-yl-carbamoyl]-5-[3-(4-fluorphenyl)-propionylamino]-pentyl-ammonium trifluoracetat (**17**),
(S)-6,8-Dichlor-3-{(S)-2-[3-(2-hydroxy-phenyl)-propionylamino]-3-methylpentanoylaminol-2,3,4,9-tetrahydro-1H-carbazol-3-carbonsäure-((S)-1-carbamoyl-2-methyl-butyl)-amid (**18**),
(R)-6,8-Dichlor-3-((S)-2-{3-[2-(2-{2-[2-(2-methoxy-ethoxy)-ethoxy]-ethoxy}-ethoxy)-phenyl]-propionylamino}-3-methyl-pentanoylamino)-2,3,4,9-tetrahydro-1H-carbazol-3-carbonsäure-((S)-1-carbamoyl-2-methyl-butyl)-amid (**20**),
(R)-6,8-Dichlor-3-((S)-2-{2-[2-(2-{2-[2-(2-methoxy-ethoxy)-ethoxy]-ethoxy}-ethoxy)-phenyl]-acetylamino}-3-methyl-pentanoylamino)-2,3,4,9-tetrahydro-1H-carbazol-3-carbonsäure-((S)-1-carbamoyl-2-methyl-butyl)-amid (**21**),
(R)-6,8-Dichlor-3-[(S)-2-[3-(2-fluor-phenyl)-propionylamino]-4-(4-hydroxy-phenyl)-butyrylamino]-2,3,4,9-tetrahydro-1H-carbazol-3-carbonsäure-((S)-1-carbamoyl-2-methyl-butyl)-amid (**22**),
(R)-6,8-Dichlor-3-{(S)-2-[3-(2-fluor-phenyl)-propionylamino]-3-methyl-pentanoylamino}-2,3,4,9-tetrahydro-1H-carbazol-3-carbonsäure-((S)-1-carbamoyl-2-methyl-butyl)-amid (**23**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-3-methyl-benzylester (**26**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-2,6-difluorbenzylester (**27**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-3,5-difluorbenzylester (**28**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-3,5-dichlorbenzylester (**29**),
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3-fluor-benzylester (**30**),
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2-fluor-benzylester (**31**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-3-chlor-benzylester (**32**),
{(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyl}-carbaminsäure-3,5-difluorbenzylester **(33),**
{(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyll-carbaminsäure-3-fluor-benzylester **(34),**
{(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyl}-carbaminsäure-2-fluor-benzylester **(35),**
[(S)-1-[(R)-3-((S)-1-Carbamoyl-2-methyl-butylcarbamoyl)-6,8-dichlor-2,3,4,9-tetrahydro-1H-carbazol-3-ylcarbamoyl]-3-(4-hydroxy-phenyl)-propyl]-carbaminsäure-3-fluor-benzylester (**37**),
[(S)-1-[(R)-3-((S)-1-Carbamoyl-2-methyl-butylcarbamoyl)-6,8-dichlor-2,3,4,9-tetrahydro-1H-carbazol-3-ylcarbamoyl]-3-(4-hydroxyphenyl)-propyl]-carbaminsäure-2-fluor-benzylester **(38),**
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-2-(2-fluor-phenyl)-ethylester **(40),**
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-3-methoxybenzylester **(47),**
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-4-fluor-benzylester (**48**),
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2-methyl-benzylester (**49**),
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2,3-dimethoxybenzylester (**50**),
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2-methoxybenzylester (**51**),
(R)-6,8-Dichlor-3-{(S)-2-[2-(2-fluor-phenyl)-ethylamino]-3-methyl-pentanoylaminol-2,3,4,9-tetrahydro-1H-carbazole-3-carbonsäure-((S)-1-carbamoyl-2-methyl-butyl)-amid **(52),**
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-2-trifluormethyl-benzylester **(53),**
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3-trifluormethyl-benzylester **(54),**
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3-trifluormethoxy-benzylester **(55),**
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2-trifluormethoxy-benzylester **(56),**
(R)-6,8-Dichlor-3-{(S)-2-[2-(4-fluor-phenyl)-ethylamino]-3-methyl-pentanoylamino}-2,3,4,9-tetrahydro-1H-carbazole-3-carbonsäure-((S)-1-carbamoyl-2-methyl-butyl)-amid **(58),**
(R)-6,8-Dichlor-3-{(S)-2-[2-(2,6-difluor-phenyl)-acetylamino]-3-methyl-pentanoylaminol-2,3,4,9-tetrahydro-1H-carbazole-3-carbonsäure-((S)-1-carbamoyl-2-methyl-butyl)-amid **(59),**
(R)-6,8-Dichlor-3-{(S)-2-[2-(3-fluor-phenyl)-ethylamino]-3-methyl-pentanoylaminol-2,3,4,9-tetrahydro-1H-carbazole-3-carbonsäure((S)-1-carbamoyl-2-methyl-butyl)-amid **(61),**
(R)-8-Chlor-6-fluor-3-{(S)-2-[2-(4-fluor-phenyl)-ethylamino]-3-methyl-pentanoylamino}-2,3,4,9-tetrahydro-1H-carbazole-3-carbonsäure-((S)-1-carbamoyl-2-methyl-butyl)-amid **(63),**
{(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyl}-carbaminsäure-4-fluor-benzylester **(64),**
(R)-8-Chlor-6-fluor-3-{(S)-2-[2-(2-fluorphenyl)-acetylamino]-3-methyl-pentanoylamino]-2,3,4,9-tetrahydro-1H-carbazol-3-carbonsäure-((S)-1-carbamoyl-2-methyl-butyl)-amid **(69),**
(R)-3-{(S)-2-[2-(2,6-Difluorphenyl)-acetylamino]-3-methyl-pentanoylamino}-8-trifluormethyl-2,3,4,9-tetrahydro-1H-carbazol-3-carbonsäure-((S)-1-carbamoyl-2-methyl-butyl)-amid (**74**),
(R)-3-{(S)-2-[2-(2-Fluorphenyl)-acetylamino]-3-methyl-pentanoylamino]-8-trifluormethyl-2,3,4,9-tetrahydro-1H-carbazol-3-carbonsäure-((S)-1-carbamoyl-2-methyl-butyl)-amid (**75**),

Die oben genannten Verbindungen **4, 30, 31, 34, 37, 48, 52, 58, 59, 61, 63, 64, 69, 74** und **75** sind dabei ganz besonders bevorzugt.

Die neuen erfindungsgemäßen Tetrahydrocarbazol-Derivate der allgemeinen Formeln (I, Ia und Ib), wie vorstehend definiert, sind Liganden von GPCRs. Somit eignen sich die vorgenannten erfindungsgemäßen Verbindungen zur Behandlung und Prophylaxe von durch einen GPCR vermittelten Krankheitszuständen, bzw. von Krankheitszuständen, die sich durch Modulation dieses Rezeptors beeinflussen und so behandeln lassen. Die erfindungsgemäßen Verbindungen können insbesondere zur Inhibierung, d.h. als Antagonisten, des LHRH-Rezeptors oder von Rezeptoren der Neurokininfamilie, insbesondere den NK₁- und/oder NK₂-Rezeptor eingesetzt werden und eignen sich somit beispielsweise zur Behandlung von gutartigen und bösartigen Tumorerkrankungen, zur Behandlung und Vorbeugung von Brechreiz und Erbrechen, zum Beispiel in der Folge emetogener Chemotherapie, zur Behandlung von Schmerzen, Entzündungen sowie rheumatischen und arthritischen Krankheitsbildern, in der männlichen Fertilitätskontrolle, zur Hormontherapie, in der Hormonersatztherapie und zur Behandlung und/oder Kontrolle weiblicher Sub- oder Infertilität.

In der männlichen Fertilitätskontrolle bewirken die erfindungsgemäßen Verbindungen eine Verringerung der Spermatogenese. Vorzugsweise erfolgt eine kombinierte Verabreichung mit Androgenen, z.B. Testosteron oder Testosteron-Derivaten, wie etwa Testosteronestern. Die Verabreichung der Testosteron-Derivate kann dabei beispielsweise durch Injektion, z.B. durch intramuskuläre Depotinjektion, erfolgen.

Auch in der weiblichen Hormontherapie, beispielsweise zur Behandlung von gutartigen hormonabhängigen Erkrankungen wie Endometriose, Uterine Fibroide, Uterus-Myome (Uterus Leiomyome), Endometrium Hyperplasie, Dysmenorrhoe, und Dysfunktionelle Uterusblutungen (Menorrhagie, Metrorrhagie) können die erfindungsgemäßen Verbindungen der allgemeinen Formeln (I, Ia und Ib) -gegebenenfalls in Kombination mit anderen Hormonen, z.B. Östrogenen oder/und Progestinen - eingesetzt werden. Besonders bevorzugt sind Kombinationen der erfindungsgemäßen LHRH-Rezeptor-Antagonisten und gewebeselektiven partiellen Östrogenagonisten wie Raloxifen^{®}.

Außerdem können die erfindungsgemäßen Verbindungen in der Hormonersatztherapie, beispielsweise zur Behandlung von Hitzewallungen, eingesetzt werden.

Darüber hinaus können die erfindungsgemäßen Verbindungen der allgemeinen Formeln (I, Ia und Ib) zur Kontrolle der weiblichen Fertilität, beispielsweise durch Ausschalten des endogenen Hormonzyklus für die kontrollierte Induzierung der Ovulation ("COS = controlled ovarien stimulation") und bei der Behandlung der Sterilität im Rahmen von künstlichen Reproduktionstechniken wie "In Vitro Fertilization" ("IVF") eingesetzt werden.

Andererseits sind die neuen, erfindungsgemäßen Verbindungen der allgemeinen Formeln (I, Ia und Ib) auch für die Empfängnisverhütung bei Frauen geeignet. So kann ein erfindungsgemäßer LHRH-Rezeptor-Antagonist an den Tagen 1 bis 15 des weiblichen Zyklus zusammen mit Östrogen, vorzugsweise mit sehr geringen Östrogendosierungen, verabreicht werden. An den Tagen 16 bis 21 des Einnahmezyklus wird Progestagen der Kombination aus Östrogen und LHRH-Rezeptor-Antagonist zugesetzt. Der erfindungsgemäße LHRH-Rezeptor-Antagonist kann kontinuierlich über die gesamte Zyklusdauer verabreicht werden. Auf diese Weise kann eine Verringerung der Hormondosierungen und somit eine Verringerung der Nebenwirkungen von unphysiologischen Hormonspiegeln erreicht werden. Weiterhin können vorteilhafte Wirkungen bei Frauen erzielt werden, die an polyzystischem Ovariensyndrom und Androgen-abhängigen Erkrankungen wie Akne, Seborrhoe und Hirsutismus leiden. Auch ist eine verbesserte Zykluskontrolle gegenüber bisherigen Verabreichungsmethoden zu erwarten.

Weitere Indikationen sind benigne Prostatahyperplasie (BPH), Gonadenprotektion bei Chemotherapie, frühkindliche Entwicklungsstörungen, z.B. Pubertas praecox, die Behandlung von HIV Infektionen bzw. AIDS und von neurologischen oder neurodegenerativen Erkrankungen, ARC (AIDS related complex), Kaposi-Sarkom, Tumoren ausgehend vom Hirn und/oder Nervensystem und/oder Hirnhäuten (vergl. WO 99/01764), Demenz und der Alzheimer Erkrankung.

Schließlich können die erfindungsgemäßen Verbindungen der allgemeinen Formeln (I, la und Ib), wie vorstehend definiert, auch für die Behandlung von malignen hormonabhängigen Tumorerkrankungen, wie prämenopausalem Brustkrebs, Prostatakrebs, Eierstockkrebs, Gebärmutterkrebs, Gebärmutterhalskrebs und Endometriumkrebs eingesetzt werden, indem sie die endogenen Sexualsteroidhormone unterdrücken, und eignen sich darüber hinaus auch zur Behandlung und Vorbeugung von Brechreiz und Erbrechen, zum Beispiel in der Folge emetogener Chemotherapie, oder zur Behandlung von Schmerzen, Entzündungen sowie rheumatischen und arthritischen Krankheitsbildern.

Die neuen, erfindungsgemäßen Verbindungen der allgemeinen Formeln (I, la und Ib), wie vorstehend definiert, sind als GPCR-Liganden, insbesondere LHRH-Rezeptor-Antagonisten oder Antagonisten von Rezeptoren der Neurokininfamilie zur Behandlung der vorstehend aufgeführten Krankheitszustände zur Verabreichung an Säugetiere und insbesondere den Menschen, jedoch auch für veterinärmedizinische Zwecke, z.B. bei Haus- und Nutztieren, aber auch bei Wildtieren geeignet.

Die Verabreichung kann auf bekannte Art und Weise, beispielsweise oral oder nicht-oral, insbesondere topisch, rektal, intravaginal, nasal oder durch Injektionen oder Implantation erfolgen. Die orale Verabreichung ist bevorzugt.

Die neuen, erfindungsgemäßen Verbindungen der allgemeinen Formeln (I, Ia und Ib) werden in eine verabreichungsfähige Form gebracht und gegebenenfalls mit pharmazeutisch verträglichen Träger- bzw. Verdünnungsmitteln vermischt. Geeignete Hilfs- und Trägerstoffe sind beispielsweise in Ullman's Encyclopedia of Technical Chemistry, Vol. 4, (1953), 1-39; Journal of Pharmaceutical Sciences, Vol. 52 (1963), 918 ff; H. v. Czetsch-Lindenwald, "Hilfsstoffe für Pharmazie und angrenzende Gebiete"; Pharm. Ind. 2, 1961, 72ff; Dr. H.P. Fiedler, "Lexikon der Hilfsstoffe für Pharmazie, Kosmetik und angrenzende Gebiete", Cantor KG, Aulendorf in Württemberg, 1971, beschrieben.

Die orale Verabreichung kann beispielsweise in fester Form als Tablette, Kapsel, Gelkapsel, Dragee, Granulat oder Pulver, jedoch auch in Form einer trinkbaren Lösung erfolgen. Zur oralen Verabreichung können die neuen, erfindungsgemäßen Verbindungen der allgemeinen Formeln (I, la und Ib), wie vorstehend definiert, mit bekannten und gewöhnlich verwendeten, physiologisch verträglichen Hilfs- und Trägerstoffen kombiniert werden, wie z.B. Gummiarabikum, Talkum, Stärke, Zucker wie z.B. Mannit, Methylcellulose, Lactose, Gelatine, oberflächenaktive Mittel, Magnesiumstearat, Cyclodextrine, wässrige oder nicht-wässrige Trägerstoffe, Verdünnungsmittel, Dispergiermittel, Emulgatoren, Schmiermittel, Konservierungsstoffe und Geschmacksstoffe (z.B. etherische Öle). Die erfindungsgemäßen Verbindungen können auch in einer mikropartikulären, z.B. nanopartikulären Zusammensetzung dispergiert sein.

Die nicht orale Verabreichung kann beispielsweise durch intravenöse, subkutane oder intramuskuläre Injektion steriler wässriger oder öliger Lösungen, Suspensionen oder Emulsionen, mittels Implantaten oder durch Salben, Cremes oder Suppositorien erfolgen. Gegebenenfalls kann auch eine Verabreichung als Retardform erfolgen. Implantate können inerte Materialen enthalten, z.B. biologisch abbaubare Polymere oder synthetische Silicone wie z.B. Silicongummi. Eine intravaginale Verabreichung kann z.B. mittels Vaginalringen erfolgen. Eine intrauterine Verabreichung kann z.B. mittels Diaphragmen oder anderer geeigneter intrauteriner Vorrichtungen erfolgen. Darüber hinaus ist auch eine transdermale Verabreichung, insbesondere mittels einer dazu geeigneten Formulierung und/oder geeigneter Mittel wie z.B. Pflaster, vorgesehen.

Wie bereits vorstehend erläutert, können die neuen, erfindungsgemäßen Verbindungen der allgemeinen Formeln (I, Ia und Ib) auch mit weiteren pharmazeutischen Wirkstoffen kombiniert werden. Im Rahmen einer Kombinationstherapie können die einzelnen wirksamen Bestandteile gleichzeitig oder getrennt verabreicht werden, und zwar entweder auf demselben Wege (z.B. oral) oder auf getrennten Wegen (z.B. oral und als Injektion). Sie können in gleichen oder unterschiedlichen Mengen in einer Einheitsdosis vorliegen bzw. verabreicht werden. Es kann auch ein bestimmtes Dosierungsregime angewendet werden, sofern dies zweckmäßig erscheint. Auf diese Weise können auch mehrere der neuen, erfindungsgemäßen Verbindungen der allgemeinen Formeln (I, Ia und Ib) miteinander kombiniert werden.

Die Dosierung kann je nach Art der Indikation, der Schwere der Erkrankung, der Art der Verabreichung, dem Alter, Geschlecht, Körpergewicht und der Sensitivität des zu behandelnden Subjekts in einem breiten Rahmen variieren. Es entspricht den Fähigkeiten eines Fachmanns, eine "pharmakologisch wirksame Menge" der kombinierten pharmazeutischen Zusammensetzung zu bestimmen. Die Verabreichung kann in einer einzigen Dosis oder mehreren getrennten Dosierungen erfolgen.

Eine geeignete Einheitsdosis ist z.B. 0,001 mg bis 100 mg des Wirkstoffs, d.h. mindestens einer erfindungsgemäßen Verbindung der allgemeinen Formeln (I, Ia und Ib) und gegebenenfalls eines weiteren Wirkstoffs, pro kg Körpergewicht eines Patienten.

In einem weiteren Aspekt der vorliegenden Erfindung sind demnach auch pharmazeutische Zusammensetzungen wie vorstehend beschrieben, umfassend eine oder mehrere der neuen erfindungsgemäßen Verbindungen der allgemeinen Formeln (I, Ia und Ib), wie vorstehend definiert, sowie gegebenenfalls pharmazeutisch verträgliche Träger- und/oder Hilfsstoffe, von der vorliegenden Erfindung erfasst. Bevorzugte und besonders bevorzugte pharmazeutische Zusammensetzungen sind jene, die mindestens eine der vorstehend genannten bevorzugten oder besonders bevorzugten neuen, erfindungsgemäßen Verbindungen der allgemeinen Formeln (I, Ia und Ib) umfassen, insbesondere mindestens eine der vorstehend namentlich genannten Verbindungen **1** bis **75**, wobei Verbindungen **4, 30, 31, 34, 37, 48, 52, 58, 59, 61, 63, 64, 69, 74** und **75** dabei ganz besonders bevorzugt sind. In pharmazeutischen Zusammensetzungen gemäß der vorliegenden Erfindung können neben mindestens einer Verbindung der allgemeinen Formeln (I, la und Ib), wie vorstehend definiert, auch noch weitere pharmazeutische Wirkstoffe vorliegen, wie vorstehend bereits näher aufgeführt.

In den erfindungsgemäßen pharmazeutischen Zusammensetzungen liegt mindestens eine der neuen, erfindungsgemäßen Verbindungen (I), wie vorstehend definiert, in einer pharmakologischen aktiven Menge, vorzugsweise in einer Einheitsdosis, z.B. der vorstehend genannten Einheitsdosis, vor, und zwar vorzugsweise in einer Verabreichungsform, die eine orale Verabreichung ermöglicht.

Darüber hinaus stellt die vorliegende Erfindung in einem weiteren Aspekt Verbindungen der allgemeinen Formeln (I, Ia und Ib) wie vorstehend definiert zur Verwendung als Arzneimittel bereit. Wie bereits vorstehend erläutert, wirken die Verbindungen der allgemeinen Formeln (I, Ia und Ib) als GPCR-Liganden, insbesondere als Antagonisten des LHRH-Rezeptors, und sind damit zur Verwendung als Arzneimittel besonders geeignet. Die erfindungsgemäßen Verbindungen der allgemeinen Formeln (I, Ia und Ib) zur Verwendung als Arzneimittel sind vorzugsweise zur Verabreichung, zur Behandlung oder Erleichterung der vorstehend genannten medizinischen Indikationen bzw. zur Empfängnisverhütung vorgesehen.

Bevorzugte erfindungsgemäße Tetrahydrocarbazol-Verbindung der allgemeinen Formeln (I, la und Ib), wie vorstehend definiert, zur Verwendung als Arzneimittel sind wiederum jene Verbindungen, die vorstehend als bevorzugte und besonders bevorzugte Verbindungen genannt wurden, insbesondere die namentlich genannten bevorzugten erfindungsgemäßen Verbindungen **1** bis **75**, sowie, sofern nicht bereits davon erfasst, die in den Beispielen genannten erfindungsgemäßen Verbindungen.

Bezüglich der erfindungsgemäßen Verbindungen der allgemeinen Formeln (I, la und Ib) umfassende pharmazeutische Zusammensetzungen sowie bezüglich der Verwendung der erfindungsgemäßen Verbindungen der allgemeinen Formeln (I, la und Ib) als Arzneimittel sei hinsichtlich Verwendungs- und Verabreichungsmöglichkeiten auf das bereits im Zusammenhang mit der Verwendung der neuen, erfindungsgemäßen Verbindungen der allgemeinen Formeln (I, Ia und Ib) selbst, Gesagte verwiesen.

In einem anderen Aspekt stellt die vorliegende Erfindung auch die Verwendung mindestens einer erfindungsgemäßen Tetrahydrocarbazol-Verbindung der allgemeinen Formeln (I, la und Ib), wie vorstehend definiert, zur Herstellung eines Arzneimittels zur Behandlung von durch GPCR vermittelten Krankheiten, bereit, wobei es sich bei dem GPCR Rezeptor bevorzugt um den LHRH Rezeptor handelt und die erfindungsgemäßen Verbindungen vorzugsweise als LHRH-Rezeptor-Antagonisten wirken.

Demgemäß stellt die vorliegende Erfindung in einem weiteren Aspekt die Verwendung mindestens einer erfindungsgemäßen Verbindung der allgemeinen Formeln (I, la und Ib) wie vorstehend definiert, oder einer entsprechenden pharmazeutischen Zusammensetzung, zur Herstellung eines Arzneimittels, das LHRH-Rezeptorantagonistisch wirkt, vorzugsweise zur Behandlung gutartiger und bösartiger Tumorerkrankungen, zur männlichen Fertilitätskontrolle, zur Hormontherapie, zur Hormonersatztherapie, zur kontrollierten ovariellen Stimulation im Rahmen einer künstlichen Befruchtung (in Vitro Fertilization), zur Behandlung und/oder Kontrolle weiblicher Sub- und Infertilität und zur weiblichen Empfängnisverhütung bereit. Hormontherapie umfasst dabei u.a. die Behandlung von Endometriose, Uterus-Leiomyomen, uterinen Fibroiden sowie benigner Prostatahyperplasie (BPH). In Bezug auf weitere Indikationen sowie Erläuterungen zu den Indikationen betreffend den gegenwärtigen Aspekt der vorliegenden Erfindungen sei auf die vorstehend gemachten Ausführungen bezüglich des ersten Aspekts der vorliegenden Erfindung, d.h. auf die erfindungsgemäßen Verbindungen der allgemeinen Formeln (I, Ia und Ib) selbst, sowie die dort gemachten Erläuterungen, verwiesen.

Die erfindungsgemäßen Verbindungen sind nicht nur zur Behandlung bzw. Therapie der genannten Krankheitszustände geeignet, sondern gleichermaßen zur Vorbeugung bzw. Prophylaxe sowie der Erleichterung (z.B. durch Unterdrückung der Symptome) dieser Krankheitszustände oder Krankheiten geeignet.

Die vorliegende Erfindung stellt in einem weiteren Aspekt die Verwendung einer erfindungsgemäßen Verbindung (1) zur Herstellung eines Arzneimittels zur Behandlung gutartiger und bösartiger Tumorerkrankungen sowie zur Hormonbehandlung bereit. Bevorzugte und besonders bevorzugte erfindungsgemäße Verbindungen für diese Verwendung sind jene Verbindungen, die bereits eingangs als bevorzugte oder besonders bevorzugte erfindungsgemäße Verbindungen der allgemeinen Formeln (I, Ia und Ib) selbst wie vorstehend definiert genannt wurden. Ganz besonders bevorzugte Verbindungen sind auch hier die vorstehend namentlich genannten Verbindungen **1** bis **75**.

Ebenfalls stellt die vorliegende Erfindung ein Verfahren zur Herstellung eines Arzneimittels zur Behandlung von durch GPCR vermittelten Krankheitszuständen bereit, wobei das Verfahren gekennzeichnet ist durch die Verwendung mindestens einer erfindungsgemäßen Verbindung der allgemeinen Formeln (I, Ia und Ib) oder einer enstprechenden pharmazeutischen Zusammensetzung. Die vorstehend gemachten Erläuterungen zu den bevorzugten und besonders bevorzugten erfindungsgemäßen Verbindungen sowie zu den speziellen Krankheitszuständen, die durch das pharmazeutische Mittel, das unter Verwendung der erfindungsgemäßen Verbindungen hergestellt wird, behandelt, vorgebeugt oder erleichtert werden können, sind auch für diesen Aspekt der vorliegenden Erfindung heranzuziehen.

Darüber hinaus stellt die vorliegende Erfindung ein Verfahren zur männlichen Fertilitätskontrolle oder zur weiblichen Empfängnisverhütung bereit, umfassend die Verabreichung einer zur männlichen Fertilitätskontrolle oder zur weiblichen Empfängnisverhütung wirksamen Menge mindestens einer erfindungsgemäßen Verbindung der allgemeinen Formeln (I, la und Ib), gegebenenfalls in Kombination mit einem weiteren Wirkstoff, an ein Subjekt, vorzugsweise ein Säugetier und besonders bevorzugt einen Menschen. Die vorstehend in Bezug auf weitere Aspekte der vorliegenden Erfindung gemachten Erläuterungen hinsichtlich bevorzugter und besonders bevorzugter erfindungsgemäßer Verbindungen sowie die Erläuterungen hinsichtlich Dosierung, Verabreichung etc. finden hier ebenfalls Anwendung.

In einem anderen Aspekt betrifft die vorliegende Erfindung ein Verfahren zur Behandlung von durch GPCR vermittelten Krankheitszuständen. Das Verfahren umfasst die Verabreichung mindestens einer erfindungsgemäßen Verbindung (I), wie vorstehend definiert, in einer pharmazeutisch wirksamen Menge an ein Säugetier und insbesondere einen Menschen, bei dem eine solche Behandlung erforderlich ist. Wie bereits vorstehend in Bezug auf die neuen, erfindungsgemäßen Verbindungen (I) sowie die erfindungsgemäßen pharmazeutischen Zusammensetzungen erläutert, obliegt es dem Fachwissen eines Fachmanns, eine pharmazeutisch wirksame Menge, abhängig von den speziellen Erfordernissen des Einzelfalls, zu bestimmen. Die bevorzugte Verabreichungsform ist die orale Verabreichung. Es ist auch vorgesehen, eine oder mehrere der erfindungsgemäßen Verbindungen (I) in Kombination mit mindestens einem weiteren Wirkstoff, wie vorstehend bereits erläutert, zu verabreichen. Die in Bezug auf die vorstehenden Aspekte der vorliegenden Erfindung gemachten Erläuterungen zu bevorzugten und besonders bevorzugten Verbindungen sowie zu den speziellen Krankheitszuständen, die behandelt, erleichtert oder vorgebeugt werden können, treffen auch auf das hier genannte Behandlungsverfahren zu. Besonders bevorzugte Verbindungen sind auch in diesem Aspekt die Verbindungen **1** bis **75**.

Darüber hinaus betrifft die vorliegende Erfindung auch ein Verfahren zur Inhibierung von GPCRs, insbesondere dem LHRH-Rezeptor oder einen Rezeptor der Neurokininfamilie in einem Patienten, umfassend die Verabreichung einer pharmazeutisch wirksamen Menge mindestens einer Verbindung der allgemeinen Formeln (I, la und Ib), wie vorstehend definiert, gegebenenfalls in Kombination mit einem weiteren Wirkstoff wie vorstehend definiert, an einen Patienten (Säugetier und insbesondere Mensch), der eine solche Behandlung benötigt. Die bevorzugten und besonders bevorzugten erfindungsgemäßen Verbindungen der allgemeinen Formeln (I, Ia und Ib) sind wiederum identisch zu den vorstehend in Bezug auf die anderen Aspekte der vorliegenden Erfindung genannten bevorzugten und besonders bevorzugten Verbindungen, insbesondere den Verbindungen **1** bis **75**. Auch die vorstehenden Erläuterungen bezüglich der Krankheitszustände, die durch Verabreichung der erfindungsgemäßen Verbindungen behandelt werden können, vorzugsweise durch deren LHRH-Rezeptor-antagonistischen Wirkung, finden für das hier beschriebene erfindungsgemäße Behandlungs-Verfahren Anwendung.

Die erfindungsgemäßen Verbindungen der allgemeinen Formeln (I, Ia und Ib), wie vorstehend definiert, können beispielsweise folgendermaßen hergestellt werden:

Zum einen können die erfindungsgemäßen Verbindungen derart synthetisiert werden, dass das abgebildete zentrale Tetrahydrocarbazolgerüst 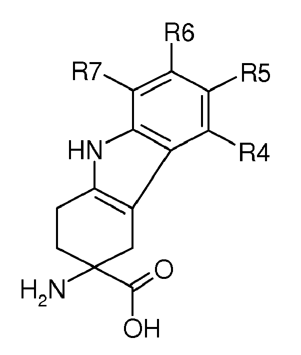 hergestellt wird, wobei dieses gegebenenfalls geschützte Tetrahydrocarbazolgerüst bereits die Substituenten R₄ bis R₇ - gegebenenfalls als Vorstufen oder in geschützter Form - enthält.

Das zentrale Tetrahydrocarbazolgerüst ist beispielsweise durch eine an sich bekannte Fischer-Indol-Synthese zugänglich. Dazu wird ein geeignet substituiertes und gegebenenfalls mit Schutzgruppen versehenes Cyclohexanonderivat mit dem jeweils gewünschten, ebenfalls geeignet substituierten und gegebenenfalls mit Schutzgruppen versehenen Phenylhydrazinderivat kondensiert (z. B. nach Britten & Lockwood, J.Chem.Soc. Perkin Trans. I 1974, 1824 oder nach Maki et al., Chem. Pharm. Bull. 1973, 21, 240). Das Cyclohexangerüst ist in der Position 4,4' durch die Reste -COOH und -NH₂ bzw. ggf. durch deren (geschützte) Vorstufen substituiert. Das Phenylhydrazingerüst ist gegebenenfalls durch die Reste R⁴ bis R⁷ substituiert. Nicht kommerziell verfügbare Phenylhydrazinderivate können durch dem Fachmann bekannte Verfahren hergestellt werden. Gegebenenfalls bei der Kondensation des Cyclohexanonderivats und des Phenylhydrazinderivats entstehende Positionsisomere können durch chromatographische Methoden wie z.B. HPLC getrennt werden.

Die Reste R₁₀R₉NCHR₈CX₃NH- und R₁R₂NCX₁CHR₃NHCX₂- können grundsätzlich nach dem Fachmann bekannten Verfahren in unterschiedlicher Weise je nach ihrer Art eingeführt und modifiziert werden, wie es etwa in der WO 03/051837 anhand von Beispielen und allgemeinen Erläuterungen aufgezeigt ist.

Ein anderes Verfahren zur Synthese der erfindungsgemäßen Verbindungen der allgemeinen Formeln (I, Ia und Ib) ist das Folgende:

Zunächst wird ein Tripeptid-Grundgerüst mittels Kupplung von drei geeigneten Aminosäuren hergestellt, wobei die erste Aminosäure AS¹ als Seitenkette den Rest R3 enthält und die dritte Aminosäure AS³ als Seitenkette den Rest R8 bzw. eine Vorstufe von R8 enthält, während die "mittlere" Aminosäure AS² 3-Amino-2,3,4,9-tetrahydro-1-H-carbazol-3-carbonsäure (abgekürzt Thc) ist. Das Thc Grundgerüst kann je nach Substitutionsmuster der gewünschten resultierenden erfindungsgemäßen Verbindung entsprechend durch die Reste R4 bis R7, gegebenenfalls in Form ihrer Vorstufen oder in geschützter Form, substituiert sein. Die Peptidkupplung kann gemäß dem Fachmann bekannter Verfahren, z.B. in der festen oder flüssigen Phase, durchgeführt werden. Anschließend können Modifikationen des Substitutionsmusters vorgenommen werden, wie das "Entschützen" bestimmter Reste.

Das folgende Schema veranschaulicht die Kopplung der Aminosäuren beispielsweise an der festen Phase: 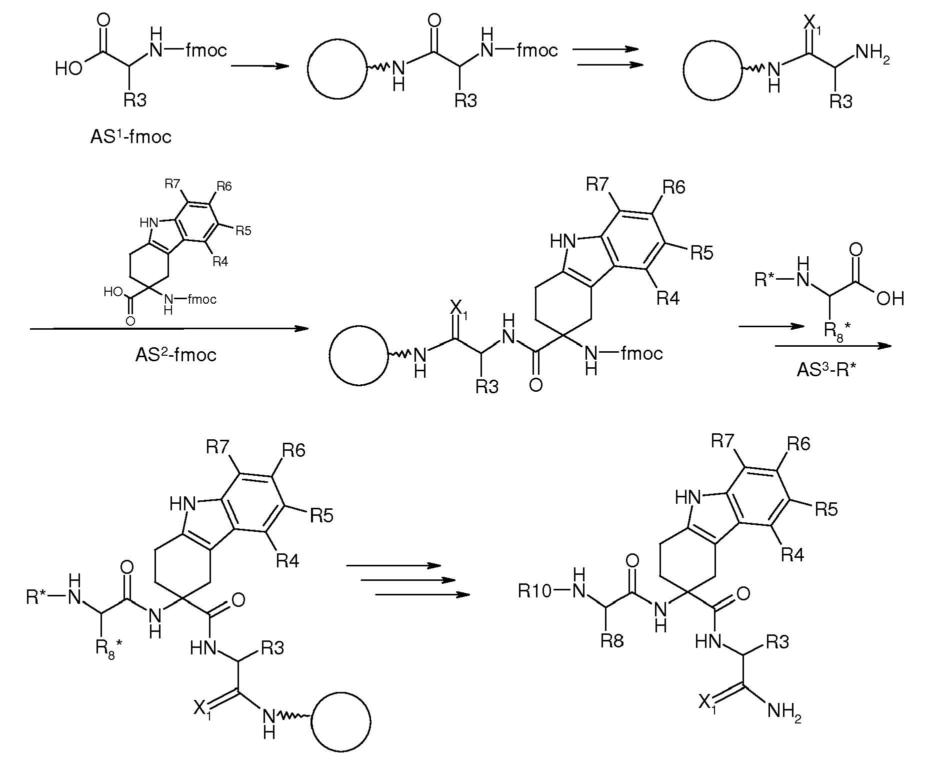 wobei R* -CO-O-benzyl (d.h. Z) oder eine allfällige Schutzgruppe, z.B. Fmoc bedeutet, wobei R8* entweder -C₁-C₆-Alkyl-Aryl oder -C₁-C₆-Alkyl-Heteroaryl bedeutet, wobei die Aryl- oder Heteroaryl-Gruppe mit bis zu drei, vorzugsweise mit einer OH-Gruppe substituiert ist, oder R8* - entsprechend der für R8 gegebenen Definitionen - auch die Bedeutung von R3 einnehmen kann

Genauer illustriert wird dieses Verfahren im Folgenden am Beispiel von Festphasen- und Flüssigphasensynthese sowie an den konkreten **Beispielen 1** bis **75**.

### Allgemeine Synthesevorschriften für die Erfindungsgemäßen Verbindungen der Allgemeinen Formeln (I, Ia und Ib)

Die Synthese der erfindungsgemäßen Verbindungen gemäß der Formeln (I, Ia und Ib) erfolgt entweder durch herkömmliche Synthese in Lösung oder aber ganz oder teilweise an der festen Phase.

### AV 1A Festphasensynthese von LHRH-Peptidomimetika

Die konkreten Synthesen der in den Beispielen aufgeführten Verbindungen erfolgten an der festen Phase mit dem halbautomatischen Synthesizer SP 650 (Fa. Labortec). Das Standardprogramm ist in nachfolgender **Tabelle 1** gezeigt:

**Tabelle 1: Programm für Synthesizer SP 650 (Firma Labortec)**

| **Schritt** | **Funktion** | **LM / Reagenz** | **Zeit in min** | **Wiederholungen** |
|---|---|---|---|---|
| 1 | waschen | DMF | 2 | 2 |
| 2 | abspalten | 20%Piperidin + 1 % DBU | 5 | 3 |
| 3 | waschen | DMF | 2 | 2 |
| 4 | waschen | 2-Propanol | 2 | 1 |
| 5 | waschen | DMF | 2 | 2 |
| 6 | waschen | 2-Propanol | 2 | 1 |
| 7 | waschen | DMF | 2 | 2 |
| 8 | STOP | | | |
| 9 | Zugabe | Fmoc-AS + HOBt + DIC | | |
| 10 | überspringen | | | |
| 11 | Kupplung | | 120 - 300 | 1 |
| 12 | waschen | DMF | 2 | 1 |
| 13 | waschen | 2-Propanol | 2 | 1 |
| 14 | waschen | DMF | 2 | 1 |
| 15 | waschen | 2-Propanol | 2 | 1 |
| 16 | waschen | DMF | 2 | 1 |
| 17 | waschen | 2-Propanol | 2 | 1 |
| 18 | ENDE | | | |

Die Abspaltung der Fmoc-Schutzgruppe erfolgt hierbei mit 20 %-igem Piperidin und 1%-igem DBU in DMF für 5 Minuten. Der Vorgang wird insgesamt dreimal für je 5 Minuten durchgeführt.

Als Waschlösungen kommen DMF und 2-Propanol zum Einsatz.

Die Kupplung der ersten Fmoc-Aminosäure erfolgt mit HOBt und DIC in DCM und DMF (v/v= 1:1).

Zur Kupplung der weiteren Fmoc-Aminosäuren wird mit HATU und HOAt (0,5 M Lösung in DMF) in Gegenwart von DIPEA gearbeitet.

### AV 1Aa Kupplung der ersten Aminosäure AS¹ (Synthese von Fmoc-AS¹-Harz)

1 mmol Fmoc-Harz [Fmoc-2,4-dimethoxy-4'-(carboxymethyloxy)-benzhydrylamin gebunden an aminomethylsubstituiertes Polystyrolharz (200 - 400 mesh; 0,55 mmol/g] nach Synthesizer-Programm vorbehandeln, Fmoc-Schutzgruppe abspalten, 1 mmol Fmoc-Aminosäure AS¹ (Fmoc-NH-CHR3-COOH), 2 mmol HOBt in DCM und DMF (v/v=1:1) und 3 mmol DIC zugeben, 3 h bei RT schütteln lassen und anschließendes Waschen nach Programm.

### AV 1Ab Kupplung der zweiten Aminosäure AS² (Synthese von Fmoc-AS²-AS¹-Harz)

1 mmol Fmoc-AS¹-Harz nach Programm vorbehandeln, 2 mmol Fmoc-Aminosäure AS² (Fmoc-Thc-OH), 2 mmol HATU, 2 mmol HOAt (0,5 m Lösung in DMF) und 5 mmol DIPEA zugeben und 4 - 6 h bei RT schütteln. Während der Reaktion pH-Wert überprüfen und durch Zugabe von DIPEA auf pH 8 - 9 einstellen. Nach beendeter Kupplung Waschschritte nach Programm.

### AV 1Ac Kupplung der dritten Aminosäure AS³ (Synthese von Z-AS³-AS²-AS¹-Harz)

1 mmol Fmoc-AS²-AS¹-Harz nach Programm vorbehandeln, 2 mmol Z-Aminosäure Z-AS³ (Benzyl-O-CO-NH-CHR8*-COOH), 2 mmol HATU, 2 mmol HOAt (0,5 m Lösung in DMF) und 5 mmol DIPEA zugeben und 4 - 6 h bei RT schütteln. Während der Reaktion pH-Wert überprüfen und durch Zugabe von DIPEA auf pH 8 - 9 einstellen. Nach beendeter Kupplung Waschschritte nach Programm.

### AV 1Ad Kupplung der dritten Aminosäure AS³ (Synthese von Fmoc-AS³-AS²-AS¹-Harz)

1 mmol Fmoc-AS²-AS¹-Harz nach Programm vorbehandeln, 2 mmol Fmoc-Aminosäure AS³ (Fmoc-NH-CHR8*-COOH), 2 mmol HATU, 2 mmol HOAt (0,5 M Lösung in DMF) und 5 mmol DIPEA zugeben und 4 - 6 h bei RT schütteln. Während der Reaktion pH-Wert überprüfen und durch Zugabe von DIPEA auf pH 8 - 9 einstellen. Nach beendeter Kupplung Waschschritte nach Programm.

### AV 1Ae Modifikation von Fmoc-AS³-AS²-AS¹-Harz: Einführung eines endständigen Z-Restes (Umsetzung mit Z-Cl)

1 mmol Fmoc-AS³-AS²-AS¹-Harz nach Programm vorbehandeln, 2 mmol Z-chlorid, 4 mmol DIPEA und katalytische Mengen an DMAP 3 h schütteln und danach Waschschritte nach Programm.

### AV 1Af Einführung eines endständigen Restes R10, wenn R10 für -CO-R11 steht - Umsetzung mit R11-COOH

1 mmol Fmoc-AS³-AS²-AS¹-Harz nach Programm vorbehandeln, 3 mmol Carbonsäure R11-COOH, 3 mmol HOBt und 4 mmol DIC für 3 h schütteln und danach Waschschritte nach Programm.

### AV 1Ag Einführung eines endständigen Restes R10, wenn R10 für -CO-OR11 steht, sowie ggf. Modifikation von R8* zu R8 - Umsetzung mit R-OSu

1 mmol Fmoc-AS³-AS²-AS¹-Harz nach Programm vorbehandeln, 3 mmol R11-OSu, 5 mmol DIPEA und katalytische Mengen an DMAP für 3 h schütteln und anschließendes Waschen nach Programm. Sofern R8* eine freie OH Gruppe aufweist, kann es zur Umsetzung dieser OH Gruppe zu -O-CO-O-R13 kommen, wobei dann R11 und R13 identische Reste sind.

### AV 1Ah Einführung eines endständigen Restes R10, wenn R10 für -R11 steht - Umsetzung mit R10-lodid (R10-I)

1 mmol Fmoc-AS³-AS²-AS¹-Harz nach Programm vorbehandeln, 1 mmol R10-1, 3 mmol Natriumhydrogencarbonat für 3 h schütteln und anschließendes Waschen nach Programm.

### AV 1Ba Einführung des Phosphorsäure-Restes an die OH-Gruppe von beispielsweise hTyr in der Seitenkette R8*

1 mmol R10-AS³-AS²-AS¹-Harz 2x mit DCM waschen, in DCM suspendieren, 2 mmol Phosphorsäure-bis-(dimethylamid)-chlorid, 2 mmol DMAP und 3 mmol DBU oder DIPEA zugeben und 4 - 6 h bei RT schütteln, und anschließend nach Programm waschen.

### AV 1Bb Einführung von Fmoc-Tyr-(PO(OBzl)-OH)-OH

1 mmol Fmoc-AS²-AS¹-Harz nach Programm vorbehandeln, 2 mmol Fmoc-Tyr(PO(OBzl)-OH)-OH, 2 mmol HATU, 2 mmol HOAt (0,5 m Lösung in DMF) und 5 mmol DIPEA zugeben und 3 h bei RT schütteln. Während der Reaktion pH-Wert überprüfen und durch Zugabe von DIPEA auf pH 8 - 9 einstellen. Nach beendeter Kupplung Waschschritte nach Programm.

### AV 2 Synthese von Thioamiden an der Festphase

Synthese erfolgt in Anlehnung an z.Bsp. H. Takuta et al. J.Org. Chem. 1989, 54, 4812 und Majer et al. Biochem & Biophys. Res. Commun. 1988, 150, 1017.

Die erste Kupplung erfolgt nach AV 1Aa.

Die Umsetzung des Carboxamids zum Thioamid erfolgt mit dem Lawesson-Reagenz in folgender Weise: 1 mmol Fmoc-AS-Harz und 2-4 mmol Lawesson- Reagenz in 20 ml getrocknetem Toluol 7 h bei 90 - 100 °C Badtemperatur rühren, das Harz absaugen und auf der Nutsche 5x im Wechsel mit DCM und warmem MeOH waschen.

Die anschließende Kupplung der zweiten und dritten Aminosäure erfolgt gemäß den Vorschriften AV 1Ab-g und AV 1B.

### AV 3A Abspaltung der Carboxamide vom Harz

Das Peptid-Harz wird vor der Abspaltung im Vakuum bei max. 40 °C getrocknet. Typischerweise werden pro Gramm Peptid 10 - 15 ml Abspaltlösung verwendet.

1 mmol Peptid-Harz in einem Gemisch aus 0,5 ml Wasser und 15 ml TFA lässt man hierzu 2 h bei 40 °C Badtemperatur rühren. Das Harz wird abgesaugt, mit wenig TFA gewaschen und die resultierende TFA-Lösung im Membranpumpenvakuum eingeengt.

Das ölige Rohprodukt wird mittels präparativer HPLC aufgereinigt - siehe AV 4.

### AV 3B Abspaltung der Thioamide vom Harz

Das Peptid-Harz wird vor der Abspaltung im Vakuum bei max. 40 °C getrocknet. Normalerweise werden pro Gramm Peptid 10 - 15 ml Spaltlösung verwendet.

Bei der Abspaltung der Thioamide ist der Zusatz von EDT als Scavenger erforderlich.

1 mmol Peptid-Harz in einem Gemisch aus 0,5 ml Wasser / 0,5 ml EDT /15 ml TFA lässt man 2 - 3 h bei 40 - 50 °C Badtemperatur rühren. Das Harz wird abgesaugt, mit wenig TFA gewaschen und die resultierende TFA-Lösung im Membranpumpenvakuum eingeengt. Das Rohprodukt wird mittels präparativer HPLC aufgereinigt - siehe AV 4.

### AV 3C Abspaltung eines Carboxamides vom Harz und gleichzeitige Hydrolyse des Phosphorsäure-bis-dimethylamid-Restes

Analog der Vorschrift AV 3A.

Zur vollständigen Hydrolyse wird nach 2 h Reaktionszeit nochmals 1 ml Wasser zugegeben und für weitere 60 min bei 40 °C rühren gelassen. Im Anschluss wird das Harz abgesaugt, mit wenig TFA gewaschen und abschließend die TFA-Lösung im Membranpumpenvakuum eingeengt. Das Rohprodukt wird mittels präparativer HPLC aufgreinigt - siehe AV 4.

### AV 3D Abspaltung eines Thioamides vom Harz und gleichzeitige Hydrolyse des Phosphorsäure-bis-(dimethylamid)-Restes

Analog der Vorschrift AV 3B.

Zur vollständigen Hydrolyse wird nach 2-3 h Reaktionszeit nochmals 1 ml Wasser zugegeben und für weitere 60 min bei 40 °C rühren gelassen. Im Anschluss wird das Harz abgesaugt, mit wenig TFA gewaschen und schließlich die TFA-Lösung im Membranpumpenvakuum eingeengt. Das Rohprodukt wird mittels präparativer HPLC aufgreinigt. - siehe AV 4.

### AV 4 Reinigung der Rohprodukte mittels semi-präparativer HPLC

Analytische und semi-präparative HPLC-Anlagen von Shimadzu; Säule 250-50, LiChrospher® 100, RP18 (12 µm) von Merck; Fluß 60 ml/min.

| | |
|---|---|
| Eluenten: | A = 970 ml Wasser + 30 ml ACN + 1 ml TFA |
| | B = 300 ml Wasser + 700 ml ACN + 1 ml TFA |

UV-Detektor 220 nm.

Alle Produkte werden durch Gradienten-Elution isoliert.

Die Rohprodukte werden in Eluent B gelöst (bei schwer löslichen Produkten Zusatz von DMF) und portionsweise über die Säule gereinigt ( z. B. 500 mg Rohprodukt in 15 ml B lösen und in einer Portion trennen). Die Trennbedingungen sind hierbei abhängig von der Peptidsequenz und Art und Menge der Verunreinigungen und werden zuvor auf der analytischen Säule experimentell ermittelt.

Ein typischer Gradient ist: 60 % B - 100 % B in 30 Minuten.

Handelt es sich bei den Rohprodukten um Diastereomerengemische, so werden sie nach dieser Methode getrennt.

Die isolierten Fraktionen werden mittels analytischer HPLC überprüft. Am Rotationsverdampfer werden ACN und TFA entfernt, das zurückbleibende wässrige Konzentrat wird lyophilisiert.

### AV 5 Flüssigphasen-Synthese von {(S)-1-[(R)-6,8-Dich)or-3-((S)-2-methy)-1-thiocarbamoyl-butylcarbamoyl)-2,3,4,9-tetrahydro-1H-carbazol-3-yl-carbamoyl]-2-methyl-butyl}-carbaminsäure-benzylester (7)

### AV 5A Synthese von ((S)-1-Carbamoyl-2-methyl-butyl)-carbaminsäure tert-butyl ester (Boc-Ile-NH₂)

10 mmol (S)-H-Ile-NH₂ Hydrochlorid wurden mit 20 mmol wässriger Soda-Lösung versetzt. Eine Lösung von 11 mmol Boc₂O in Dioxan wurde langsam bei RT zur wässrigen Lösung zugetropft und das Reaktionsgemisch für weitere 60 min bei RT gerührt. Das ausgefallene Rohprodukt wurde anschließend abgesaugt, in Wasser suspendiert und durch Zutropfen von 20 %iger Salzsäure pH-sauer gestellt. Das Rohprodukt wurde erneut abgenutscht, mit Wasser gewaschen und bei 50 °C im Vakuum über P₄O₁₀ getrocknet.
Ausbeute 85 %, Fp 167 °C (Soll 166 °C)

### AV 5B Synthese von ((S)-2-Methyl-1-thiocarbamoyl-butyl)-carbaminsäure tert-butyl ester (Boc-lle-thioamid)

Die Synthese erfolgte in Anlehnung an z. B. H. Takuta et al. J.Org. Chem. 1989, 54, 4812 und Majer et al. Biochem & Biophys. Res. Commun. 1988, 150, 1017.

10 mmol (S)-Boc-lle-NH2 wurden in 50 ml THF suspendiert, 6 mmol Lawesson-Reagenz zugegeben und 20 h bei RT gerührt. Aus der Suspension entstand eine klare Lösung. Die Reaktionslösung wurde schließlich im Membranpumpenvakuum eingeengt.

Die Aufreinigung des Rohproduktes erfolgte säulenchromatographisch (DCM + Ethylacetat = 9:1)
Ausbeute 88,9 %, Fp 131 °C (Soll 132 °C)

### AV 5C Synthese von (S)-2-Amino-3-methyl-pentansäureamid (H-Ile-thioamid)

10 mmol (S)-Boc-Ile-thioamid wurden in 40 ml DCM und 10 ml TFA 4 h bei RT gerührt. Die Reaktionslösung wurde schließlich im Membranpumpenvakuum eingeengt, der resultierende Rückstand mit 50 ml Wasser versetzt, mit konz. Ammoniak-Lösung auf einen pH-Wert von 8 eingestellt und abschließend 5 x mit Ethylacetat ausgeschüttelt. Die vereinigten, organischen Extrakte wurden mit gesättigter Natriumchlorid-Lösung gewaschen, über Natriumsulfat getrocknet, filtriert und das Filtrat im Membranpumpenvakuum eingeengt.
Ausbeute 87,5 % (gelber Feststoff)

### AV 5D Synthese von (R/S)-3-((S)-2-Benzyloxycarbonylamino-3-methylpentanoylamino)-6,8-dichlor-2,3,4,9-tetrahydro-1H-carbazol-3-carbonsäure (Z-(S)-Ile-(R/S)-(6,8-Cl)-Thc-OH)

10 mmol (R/S)-H-(6,8-Cl)-Thc, 12 mmol (S)-Z-Ile-OSu, 30 mmol DIPEA und eine Spatelspitze DMAP wurden in 50 ml DMF gegeben und 4 h bei 80 °C Badtemperatur gerührt. Das Reaktionsgemisch wurde anschließend im Membranpumpenvakuum eingeengt, der Rückstand mit Wasser versetzt, mit verdünnter Salzsäure ansgesäuert und mit Ethylacetat extrahiert. Die organische Phase wurde anschließend mit gesättigter Natriumchlorid-Lösung gewaschen, über Natriumsulfat getrocknet, abfiltriert, und das Filtrat wurde im Membranpumpenvakuum eingeengt.
Ausbeute 11,9 g Diastereomerengemisch (1:1 Gemisch)

### AV 5E Synthese von {(S)-1-[(R)-6,8-Dich)or-3-((S)-2-methy)-1-thiocarbamoyl-butylcarbamoyl)-2,3,4,9-tetrahydro-1H-carbazol-3-yl-carbamoyl]-2-methyl-butyl}-carbaminsäure-benzylester (7) ((S)-Z-Ile-(R)-(6,8-Cl)-Thc-(S)-Ile-thioamid)

3 mmol Z-Ile-(R/S)-(6,8-Cl)-Thc-OH, 3 mmol H-(S)-Ile-thioamid, 3 mmol HATU und 15 mmol DIPEA wurden in 5 ml DMF 3 min bei max. 100 °C und max. 150 Watt in der Mikrowelle erhitzt. Die Reaktionslösung wurde mit Eluens B verdünnt und in 2 Portionen über die präparative HPLC-Säule getrennt (siehe AV4).
Ausbeute Diastereomer 1 = 19 % HPLC-Reinheit 98,5 % (Verbindung 7)
Ausbeute Diastereomer 2 = 17,7 % HPLC-Reinheit 95 %

### AV 6 Beispielhafte Synthese von C-terminalen, substituierten Amiden in Lösung (S)-2-{[(R/S)-3-((S)-2-Benzyloxycarbonylamino-3-methyl-pentanoylamino)-6,8-dichlor-2,3,4,9-tetrahydro-1H-carbazol-3-carbonyl]-amino}-3-methylpentansäure ((S)-Z-lle-(R/S)-(6,8-Cl)-Thc-(S)-Ile-OH) + R1-NH-R2

1 mmol (S)-Z-Ile-(R/S)-(6,8-Cl)-Thc-(S)-Ile-OH (*) werden in 5 ml DMF suspendiert, 1,1 mmol R1-NH-R2, 1,2 mmol PyBOP und 3 mmol NMM werden hinzugefügt und 16 h bei RT rühren gelassen. Das Reaktionsgemisch wird im Membranpumpenvakuum eingeengt und mittels präparativer HPLC (siehe AV4) aufgereinigt. Als Produkt erhält man in diesem Fall (S)-Z-Ile-(R/S)-(6,8-Cl)-Thc-(S)-Ile-NR1R2

(*) Die Synthese von (S)-Z-Ile-(R/S)-(6,8-Cl)-Thc-(S)-Ile-OH kann über Festphase mit 2-Chlortritylchlorid-Harz (1,37 mmol/g - Alexis Biochemicals 120-002-0000) durchgeführt werden. Die erste Kupplung wird in DCM in Gegenwart von DIPEA, die zweite und dritte Kupplung analog AV 1Ab + 1Ac - 1Ag durchgeführt. Die Abspaltung erfolgt gemäß AV 3A, die Reinigung gemäß AV 4.

### AV 7 Umsetzung von Lawesson Reagenz mit R10-AS³-AS²-AS'-NH₂ Alternative Methode zur Thiierung von Sequenzen mit C-terminaler Amidfunktion wenn AS² eine Thc-Baustein ist

1 mmol R10-AS³-AS²-AS¹-NH₂ werden in 40 ml getrocknetem Toluol gelöst, 1 mmol Lawesson-Reagenz bei RT wird zugegeben, die Suspension 3 - 4 h bei 80 °C Badtemperatur gerührt, und die Reaktionslösung im Membranpumpenvakuum eingeengt. Den Rückstand trennt man mittels präparativer HPLC analog AV 4 auf.

### AV 8 Darstellung von Alkyl-Aryl-Ethern durch Mitsunobu Reaktion

Alkyl-Aryl-Ether werden aus entsprechenden OH-Verbindungen unter Zusatz von PPh₃ und DEAD hergestellt (Mitsunobu et al., J. Am. Chem. Soc. 1972, 94, 679) hergestellt.

Im Folgenden wird nochmals die allgemeine Herstellungsweise für die erfindungsgemäßen Erfindungen unter Angabe der entsprechenden Verfahrensschritte und Arbeitsvorschriften zusammengefasst:
a) Zunächst wird die Fmoc geschützte AS¹ gemäß AV 1Aa an das Harz gekoppelt, 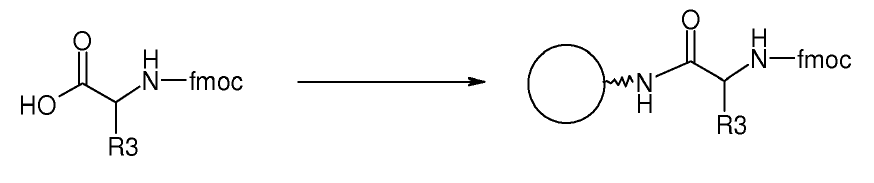
b) ggf. erfolgt in einem zweiten Schritt gemäß AV2 die Umwandlung in ein Thioamid 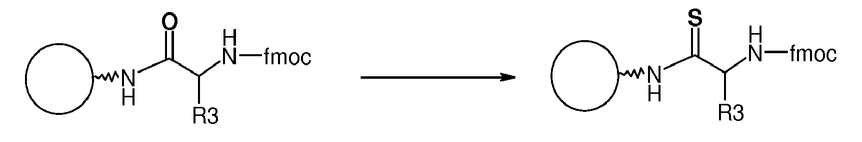
c) Das aus Schritt a oder b erhaltene Produkt wird nach Abspaltung der Schutzgruppe mit der geschützten Aminosäure 2, dem entsprechend substituierten Thc-Derivat, umgesetzt gemäß AV 1 Ab: 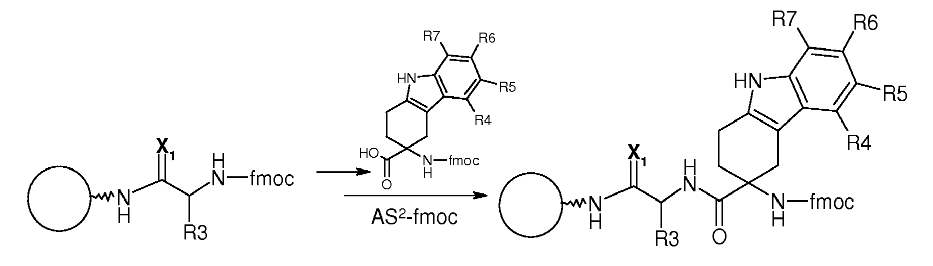
d) Dann wird das aus Schritt c erhaltene Produkt, nach der Abspaltung der Schutzgruppe, mit der dritten geschützten AS umgesetzt: 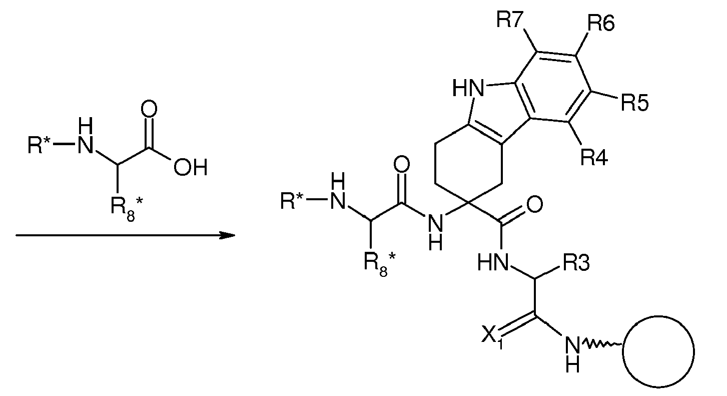 wobei R* = Z = -CO-O-benzyl (gemäß AV 1Ac) oder R* = Fmoc (gemäß AV 1Ad) ist, und R8* -C₁-C₆-Alkyl-Aryl oder -C₁-C₆-Alkyl-Heteroaryl bedeutet, wobei die Aryl- oder Heteroaryl-Gruppe mit bis zu drei, vorzugsweise mit einer OH-Gruppe substituiert ist, oder R8* - entsprechend der für R8 gegebenen Definitionen - auch die Bedeutung von R3 einnehmen kann.
e) Sofern R* = Fmoc wird das im Schritt d erhaltene Produkt zunächst entschützt und die endständige freie Aminogruppe anschließend so umgesetzt, dass der Rest R10 eingeführt wird: 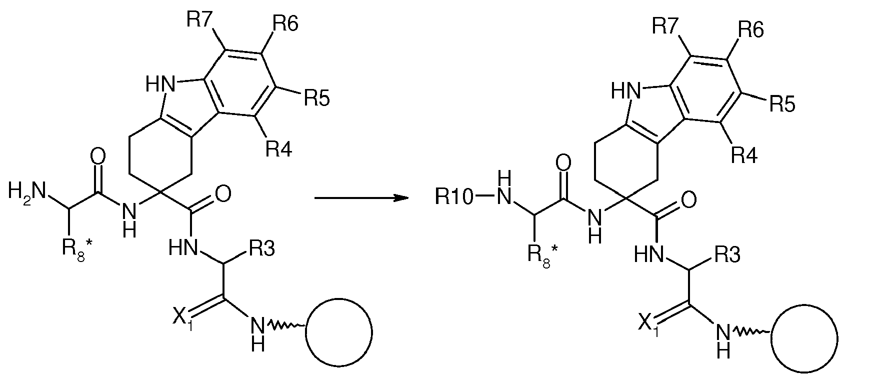 Je nach Natur von R10 werden unterschiedliche Verfahren angewendet:
   (i) AV 1Af für R10 = -CO-R11; umgesetzt wird mit R11-COOH
   (ii) AV 1Ag für R10 = -CO-OR11; umgesetzt wird mit R11-OSu; hier kann es, sofern R8* eine freie OH Gruppe aufweist, ebenfalls zur Umsetzung dieser OH Gruppe zu -O-CO-O-R13 kommen, wobei dann R11 und R13 identische Reste sind
   (iii) AV 1Ah für R10 = -R11; umgesetzt wird mit R10-lodid (R10-I)
f) Im optionalen Schritt f kann ggf. noch die Umwandlung von R8* in R8 erfolgen, z.B. durch
   (i) Einführung eines Phosphorsäure-Restes in R8 durch Umwandlung einer OH Gruppe gemäß Schritt AV 1Ba; oder
   (ii) Einführung eines -CO-O-benzyl Restes (Z) in R8 durch Umwandlung einer OH Gruppe gemäß Schritt AV 1Ae;
g) Dann folgt die Abspaltung des Tripeptids vom Harz gemäß einer der Vorschriften AV 3A, 3B, 3C oder 3D, sowie die Aufreinigung nach Vorschrift AV 4.
h) Sofern noch weitere Modifikationen von R8 oder R11 erforderlich sind, können diese jetzt erfolgen, z.B. die Einführung von Alkyl-Ethern aus den entsprechenden OH-Verbindungen gemäß Schritt AV8.

Die Darstellung der in den **Beispielen 1** bis **6** und **16** bis **75** genannten erfindungsgemäßen Verbindungen erfolgte wie nachstehend genauer angegeben gemäß der Arbeitsvorschriften AV 1-8 wie vorstehend definiert. Die analytische Charakterisierung der erfindungsgemäßen Verbindungen erfolgte ¹H-NMR spektroskopisch und/oder massenspekrometrisch.

Die eingesetzten Chemikalien und Lösungsmittel wurden kommerziell bei den herkömmlichen Anbietern erworben (Acros, Avocado, Aldrich, Bachem, Fluka, Lancaster, Maybridge, Merck, Sigma, TCI etc.) oder nach dem Fachmann bekannten Verfahren synthetisiert.

Für die nachfolgend aufgezeigten Ausführungsbeispiele wurden chirale Bausteine in der Regel in enantiomerenreiner Form zum Einsatz gebracht. Im Falle des Tetrahydrocarbazoleduktes kam der racemische Baustein zum Einsatz. Endprodukte wurden durch semi-präparative HPLC gereinigt und in diastereomerenreiner Form charakterisiert.

Die Benennung der erfindungsgemäßen Verbindungen der allgemeinen Formeln (I, la und Ib), insbesondere der Verbindungen **1** bis **75**, erfolgte mit der AutoNom 2000 - Software (ISIS^{™}/ Draw 2.5; MDL).

Die Erfindung soll anhand der nachfolgenden Beispiele näher erläutert werden, ohne jedoch auf diese Beispiele beschränkt zu sein.

### Liste verwendeter Abkürzungen:

- z.Bsp.: zum Beispiel
- DBU: 1,8-Diazabicyclo[5.4.0]undec-7-en
- HOBt: 1-Hydroxybenzotriazol
- Fmoc: 9-Fluorenylmethoxycarbonyl
- Boc: *tert*.-Butyloxycarbonyl
- Z: Benzyloxycarbonyl
- Z-Cl: Benzyloxycarbonylchlorid
- Boc₂O: Di-tert.-butyldicarbonat
- Bzl: Benzyl
- AS: Aminosäure
- EDT: 1,2-Ethandithiol
- DEAD: Diethylazodicarboxylat
- DIC: N,N'-Diisopropylcarbodiimid
- DCC: N,N'-Dicyclohexylcarbodiimid
- HATU: N,N,N',N'-Tetramethyl-O-(7-azabenzotriazol-1-yl)uronium hexafluorophosphat
- HOAt: 1-Hydroxy-7-azabenzotriazol
- PyBop: (Benzotriazol-1-yloxy)tripyrrolidinophosphonium hexafluorophosphat
- OSu: N-Hydroxysuccinimidyl
- DIPEA: Diisopropylethylamin
- DMAP: N,N'-Dimethylaminopyridin
- DBU: 1,8-Diazabicyclo[5.4.0]undec-7-en
- NMM: N-Methylmorpholin
- TFA: Trifluoressigsäure
- DCM: Dichlormethan
- DMF: N,N'-Dimethylformamid
- DMA: N,N'-Dimetylacetamid
- ACN: Acetonitril
- THF: Tetrahydrofuran
- Me: Methyl
- MeOH: Methanol
- Lawesson-Reagenz: 2,4-Bis(4-methoxyphenyl)-1,3-dithia-2,4-diphosphetan-2,4disulfid
- Thc: 3-Amino-2,3,4,9-tetrahydro-1 H-carbazol-3-carbonsäure
- Ala: Alanin(yl)
- Val: Valin(yl)
- Ile: Isoleucin(yl)
- Leu: Leucin(yl)
- Gln: Glutamin(yl)
- Asn: Asparagin(yl)
- Tyr: Tyrosin(yl)
- hTyr: homo-Tyrosin(yl)
- Arg: Arginin(yl)
- Lys: Lysin(yl)
- RT: Raumtemperatur
- Fp: Festpunkt (Schmelzpunkt)
- ml: Milliliter
- min: Minute
- h: Stunde
- ELISA: Enzyme Linked Immunosorbent Assay
- HEPES: N-(2-hydroxyethyl)piperazin-N'-2-ethansulfonsäure
- DMEM: Dulbecco's Modified Eagles Medium
- RIA: Radio Immuno Assay
- LHRH: Luteinizing Hormone Releasing Hormone
- LH: Luteinizing Hormone
- NK1: Neurokinin 1
- NK2: Neurokinin 2

### Beispiele

### I. Synthese Erfindungsgemäßer Verbindungen

### Beispiel 1:

### {(S)-1-[(R)-3-((S)-1-Carbamoyl-2-methyl-propylcarbamoyl)-6,8-dichlor-2,3,4,9-tetrahydro-1H-carbazo1-3-yl-carbamoyl]-2-methyl-propyl}-carbaminsäure-4-chlor-benzylester (1)

Gemäß den allgemeinen Arbeitsvorschriften AV 1Aa, b, d, g, 3A und 4 wurden ausgehend von 3 mmol Harz, Fmoc-Val-OH (AS¹ und AS³), Fmoc-(6,8-Dichlor)-Thc-OH (AS²) und Kohlensäure-4-chlorbenzylester-2,5-dioxo-pyrrolidin-1-yl-ester 0,275 g an 1 erhalten.
Ausbeute: 0,275 g (14,44 % d. Th.)
¹H-NMR (DMSO-d6, 300K, 500 MHz):
δ =11.33 (s, 1H); 7.40-7.02 (m, 10H); 4.94 (d, 1H); 4.75 (d, 1H); 4.15 (dd, 1H); 3.87 (dd, 1H); 3.02 (d, 1H); 2.87 (d, 1H); 2.82-2.68 (m, 2H); 2.12 (m, 1H); 1.95 (m, 1H); 1.87 (m, 1H); 0.88-0.72 (m, 12H) ppm
ESI-MS: gefunden: 664.1 (M+H⁺) / kalkuliert: 663g/mol

### Beispiel 2:

### {(S)-1-[(R)-3-((S)-1-Carbamoyl-2-methyl-propylcarbamoyl)-6,8-dichlor-2,3,4,9-tetrahydro-1H-carbazol-3-yl-carbamoyl]-2-methyl-butyl}-carbaminsäure-4-chlor-benzylester (2)

Gemäß den allgemeinen Arbeitsvorschriften AV 1Aa, b, d, g, 3A und 4 wurden ausgehend von 2,5 mmol Harz, Fmoc-Val-OH (AS¹), Fmoc-Ile-OH (AS³), Fmoc-(6,8-Dichlor)-Thc-OH (AS²) und Kohlensäure-4-chlorbenzylester-2,5-dioxo-pyrrolidin-1-yl-ester 0,190 g an **2** erhalten.
Ausbeute: 0,190 g (10,64 % d. Th.)
¹H-NMR (DMSO-d6, 300K, 500 MHz):
δ =11.33 (s, 1H); 7.38-7.03 (m, 9H); 4.94 (d, 1H); 4.7 (d, 1H); 4.15 (dd, 1H); 3.87 (dd, 1H); 2.98 (d, 1H); 2.88 (d, 1H); 2.78-2.55 (m, 3H); 2.1 (m, 1H); 1.95 (m, 1H); 1.62 (m, 1H); 1.33 (m, 1H); 1.03 (m, 1H); 0.75 (m, 12H) ppm
ESI-MS: gefunden: 678.2 (M+H⁺) / kalkuliert: 677g/mol

### Beispiel 3:

### {(S)-1-[(R)-3-((S)-Carbamoyl-2-methyl-butylcarbamoyl)-6,8-dichlor-2,3,4,9-tetrahydro-1H-carbazol-3-yl-carbamoyl]-2-methyl-butyl}-carbaminsäure-4-chlor-benzyleste (3),

Gemäß den allgemeinen Arbeitsvorschriften AV 1Aa, b, d, g, 3A und 4 wurden ausgehend von 2,5 mmol Harz, Fmoc-lle-OH (AS¹ und AS³), Fmoc-(6,8-Dichlor)-Thc-OH (AS²) und Kohlensäure-4-chlorbenzylester-2,5-dioxo-pyrrolidin-1-yl-ester 0,155 g an 3 erhalten.
Ausbeute: 0,155 g (8,85 % d. Th.)
¹H-NMR (DMSO-d6, 300K, 500 MHz):
δ =11.35 (s, 1H, Indol-NH); 7.4-7.03 (3m, 4H, 3H, 3H); 4.96, 4.7 (2d, 2H, C₅H₆-CH₂); 4.18 (dd, 1H); 3.89 (dd, 1H); 2.98, 2.88 (2d, 2H, CH₂); 2.77, 2.71, 2.62, 2.12 (4m, 4H, CH₂-CH₂) 1.82 (m, 1H); 1.64 (m, 1H); 1.45-1.3 (m, 2H); 1.05 (m, 2H); 0.84 (d, 3H, CH3); 0.82 (d, 3H, CH₃); 0.79 (t, 3H, CH₃); 0.73 (t, 3H, CH₃) ppm
ESI-MS: gefunden: 692.2 (M+H⁺) / kalkuliert: 691. g/mol

### Beispiel 4:

### (R)-6,8-Dichlor-3-{(S)-2-[2-(2-fluorphenyl)-acetylamino]-3-methyl-pentanoylamino}-2,3,4,9-tetrahydro-1H-carbazol-3-carbonsäure ((S)-1-carbamoyl-2-methylbutyl)-amid (4)

Gemäß den allgemeinen Arbeitsvorschriften AV 1Aa, b, d, f, 3A und 4 wurden ausgehend von 7,0 mmol Harz, Fmoc-Ile-OH (AS¹ und AS³), Fmoc-(6,8-Dichlor)-Thc-OH (AS²) und 2-Fluorphenylessigsäure 0,995 g an **4** erhalten.
Ausbeute: 0,995 g (19,64% d. Th.)
1H-NMR (DMSO-d6, 300K, 600 MHz):
δ =11.35 (s, 1H, Indol-NH); 8.12 (s, 1H ); 7.82 (d, 1H); 7.40 (s, 1H); 7.29-7.23 (m, 2H); 7.20-7.13 (m, 3H); 7.12-7.06 (m, 3H); 4.12 (m, 2H); 3.47, 3.21 (2d, 2H, CH2); 2.99, 2.94 (2d, 2H, CH2); 2.79, 2.68 (2m, 2H, CH2); 2.59 (m, 1H); 2.12 (m, 1H); 1.62 (m, 2H); 1.33 (m, 2H); 1.01 (m, 2H); 0.80-0.71 (4m, 12H, 4 CH3) ppm
ESI-MS: gefunden: 660.3 (M+H⁺) / kalkuliert: 659.251g/mol

### Beispiel 5:

### (R)-6,8-Dichlor-3-{(S)-2-[2-(3-fluorphenyl)-acetylamino]-3-methyl-pentanoylaminol-2,3,4,9-tetrahydro-1H-carbazol-3-carbonsäure-((S)-1-carbamoyl-2-methyl-butyl)-amid (5)

Gemäß den allgemeinen Arbeitsvorschriften AV 1Aa, b, d, f, 3A und 4 wurden ausgehend von 3,0 mmol Harz, Fmoc-Ile-OH (AS¹ und AS³), Fmoc-(6,8-Dichlor)-Thc-OH (AS²) und 3-Fluorphenylessigsäure 0,185 g an 5 erhalten.
Ausbeute: 0,185 g (11% d. Th.)
¹H-NMR (DMSO-d6, 300K, 500 MHz):
δ =11.2 (s, 1H, Indol-NH); 8.17 (d, 1H, NH); 8.07 (s, 1H, NH); 7.36 (s, 1H); 7.35-7.22 (m, 2H); 7.12-7.0 (m, 6H); 4.12-4.07 (m, 2H) ; 3.55, 3.45 (2d, 2H, C₆H₅F-CH₂); 3.4, 3.0 (2d, 2H, CH₂, Thc); 2.85 (m, 1H); 2.75-2.68 (m, 1H); 2.35-2.28 (m, 1H); 2.1-2.0 (m, 1H); 1.65 (m, 1H); 1.45 (m, 1H); 1.32 (m, 1H); 1.15-1.0 (m, 2H); 0.82-0.7 (m, 7H); 0.5-0.4 (d, t, 6H, CH₃) ppm.
ESI-MS: gefunden: 660.3 (M+H⁺) / kalkuliert: 659. g/mol

### Beispiel 6:

### {(S)-1-[(R)-3-((S)-Carbamoyl-2-methyl-butylcarbamoyl)-6,8-dichlor-2,3,4,9-tetrahydro-1H-carbazol-3-yl-carbamoyl]-2-methyl-butyl}-carbaminsäure-2-chlor-benzylester (6),

Gemäß den allgemeinen Arbeitsvorschriften AV 1Aa, b, d, g, 3A und 4 wurden ausgehend von 3,0 mmol Harz, Fmoc-lle-OH (AS¹ und AS³), Fmoc-(6,8-Dichlor)-Thc-OH (AS²) und Kohlensäure-2-chlorbenzylester-2,5-dioxo-pyrrolidin-1-yl-ester 0,255 g an 6 erhalten.
Ausbeute: 0,255 g (14,72% d. Th.)
¹H-NMR (DMSO-d6, 300K, 500 MHz):
δ =11.35 (s, 1H, Indol-NH); 7.75 (s, 1H, NH); 7.4 (d, 1H, NH); 7.35-7.2 (m, 6H); 7.1-7.05 (m, 2H); 5.05, 4.82 (2d, 2H, C₅H₆-CH₂); 4.18 (dd, 1H); 3.9 (dd, 1H); 2.98, 2.88 (2d, 2H, CH₂); 2.8, 2.72, 2.62, 2.12 (4m, 4H, CH₂-CH₂) 1.75 (m, 1H); 1.62 (m, 1H); 1.45-1.25 (m, 2H); 1.05 (m, 2H); 0.68(m, 12H, CH₃) ppm.
ESI-MS: gefunden: 692.2 (M+H⁺) / kalkuliert: 691. g/mol

Daten zu weiteren Ausführungsbeispielen sind in der folgenden **Tabelle 2** zusammengefasst:

**Tabelle 2: Ausführungsbeispiele mit Synthesefolge und MS-Daten**

| **Nr.** | **Name gemäß AUTONOM** | **Synthese gemäß** | **ESI-MS kalkuliert** | **ESI-MS gefunden (M+H⁺)** |
|---|---|---|---|---|
| **16** | (R)-6,8-Dichlor-3-{(S)-2-[3-(4-fluorphenyl)-propionylamino]-3-methyl-pentanoylamino}-2,3,4,9-tetrahydro-1H-carbazol-3-carbonsäure-((S)-1-carbamoyl-2-methyl-propyl)-amid | AV: 1Aa, b, d, f, 3A, 4 | 659 | 660,3 |
| **17** | (S)-5-[(R)-3-((S)-1-Carbamoyl-2-methyl-propylcarbamoyl)-6,8-dichlor-2,3,4,9-tetrahydro-1H-carbazol-3-yl-carbamoyl]-5-[3-(4-fluorphenyl)-propionylamino]-pentyl-ammonium trifluoracetat | AV: 1Aa, b, d, f, 3A, 4 | 674 | 675,3 |
| **18** | (S)-6,8-Dichlor-3-{(S)-2-[3-(2-hydroxy-phenyl)-propionylamino]-3-methyl-pentanoylamino}-2,3,4,9-tetrahydro-1H-carbazol-3-carbonsäure-((S)-1-carbamoyl-2-methyl-butyl)-amid | AV: 1Aa, b, d, f, 3A, 4 | 671 | 672,3 |
| **20** | (R)-6,8-Dichlor-3-((S)-2-{3-[2-(2-{2-[2-(2-methoxy-ethoxy)-ethoxy]-ethoxy}-ethoxy)-phenyl]-propionylamino}-3-methyl-pentanoylamino)-2,3,4,9-tetrahydro-1H-carbazol-3-carbonsäure-((S)-1-carbamoyl-2-methyl-butyl)-amid | AV: 1Aa, b, d, f, 3A, 4, 8, 4 | 861 | 862,4 |
| **21** | (R)-6,8-Dichlor-3-((S)-2-{2-[2-(2-{2-[2-(2-methoxy-ethoxy)-ethoxy]-ethoxy}-ethoxy)-phenyl]-acetylamino)-3-methyl-pentanoylamino)-2,3,4,9-tetrahydro-1H-carbazol-3-carbonsäure-((S)-1-carbamoyl-2-methyl-butyl)-amid | AV: 1Aa, b, d, f, 3A, 4, 8, 4 | 847 | 848,4 |
| **22** | (R)-6,8-Dichlor-3-[(S)-2-[3-(2-fluor-phenyl)-propionylamino]-4-(4-hydroxy-phenyl)-butyrylamino]-2,3,4,9-tetrahydro-1H-carbazol-3-carbonsäure-((S)-1-carbamoyl-2-methyl-butyl)-amid | AV: 1Aa, b, d, f, 3A, 4 | 737 | 738,3 |
| **23** | (R)-6,8-Dichlor-3-1(S)-2-[3-(2-fluor-phenyl)-propionylamino]-3-methyl-pentanoylaminol-2,3,4,9-tetrahydro-1H-carbazol-3-carbonsäure-((S)-1-carbamoyl-2-methyl-butyl)-amid | AV: 1Aa, b, d, f, 3A, 4 | 673 | 674,4 |
| **26** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3-methyl-benzylester | AV: 1Aa, b, d, g, 3A, 4 | 671 | 672.2 |
| **27** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2,6-difluor-benzylester | AV: 1Aa, b, d, g, 3A, 4 | 693 | 694.3 |
| **28** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3,5-difluor-benzylester | AV: 1Aa, b, d, g, 3A, 4 d, g, 3A, 4 | 693 | 694.4 |
| **29** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3,5-dichlor-benzylester | AV: 1Aa, b, d, g, 3A, 4 d, g, 3A, 4 | 725 | 726.2 |
| **30** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3-fluor-benzylester | AV: 1Aa, b, d, g, 3A, 4 d, g, 3A, 4 | 675 | 676.4 |
| **31** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2-fluor-benzylester | AV: 1Aa, b, d, g, 3A, 4 d, g, 3A, 4 | 675 | 676.4 |
| **32** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3-chlor-benzylester | AV: 1Aa, b, d, g, 3A, 4 | 691 | 694.3 |
| **33** | {(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyl}-carbaminsäure-3,5-difluorbenzylester | AV: 1Aa, b, d, g, 3A, 4 | 677 | 678.3 |
| **34** | {(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyl}-carbaminsäure-3-fluor-benzylester | AV: 1Aa, b, d, g, 3A, 4 d, g, 3A, 4 | 659 | 660.4 |
| **35** | {(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyl}-carbaminsäure-2-fluor-benzylester | AV: 1Aa, b, d, g, 3A, 4 | 659 | 660.4 |
| **37** | [(S)-1-[(R)-3-((S)-1-Carbamoyl-2-methyl-butylcarbamoyl)-6,8-dichlor-2,3,4,9-tetrahydro-1H-carbazol-3-ylcarbamoyl]-3-(4-hydroxy-phenyl)-propyl]-carbaminsäure-3-fluor-benzylester | AV: 1Aa, b, d, g, 3A, 4 d, g, 3A, 4 | 739 | 740.4 |
| **38** | [(S)-1-[(R)-3-((S)-1-Carbamoyl-2-methyl-butylcarbamoyl)-6,8-dichlor-2,3,4,9-tetrahydro-1H-carbazol-3-ylcarbamoyl]-3-(4-hydroxyphenyl)-propyl]-carbaminsäure-2-fluor-benzylester | AV: 1Aa, b, d, g, 3A, 4 | 739 | 740.3 |
| **40** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl]-carbaminsäure-2-(2-fluor-phenyl)-ethylester | AV: 1Aa, b, d, g, 3A, 4 d, g, 3A, 4 | 689 | 690.3 |
| **47** | {(S)-1-1(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3-methoxy-benzylester | AV: 1Aa, b, d, g, 3A, 4 | 687 | 688.3 |
| **48** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl]-carbaminsäure-4-fluor-benzylester | AV: 1Aa, b, d, g, 3A, 4 d, g, 3A, 4 | 675 | 676.4 |
| **49** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2-methyl-benzylester | AV: 1Aa, b, d, g, 3A, 4 | 671 | 672.3 |
| **50** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2,3-dimethoxy-benzylester | AV: 1Aa, b, d, g, 3A, 4 | 717 | 718.3 |
| **51** | ((S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2-methoxy-benzylester | AV: 1Aa, b, d, g, 3A, 4 d, g, 3A, 4 | 687 | 688.3 |
| **52** | (R)-6,8-Dichlor-3-{(S)-2-[2-(2-fluor-phenyl)-ethylamino]-3-methyl-pentanoylamino}-2,3,4,9-tetrahydro-1H-carbazole-3-carbonsäure-((S)-1-carbamoyl-2-methyl-butyl)-amid | AV: 1Aa, b, d, h, 3A, 4 | 645 | 646.3 |
| **53** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2-trifluormethyl-benzylester | AV: 1Aa, b, d, g, 3A, 4 d, g, 3A, 4 | 725 | 726.4 |
| **54** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl]-carbaminsäure-3-trifluormethyl-benzylester | AV: 1Aa, b, d, g, 3A, 4 | 725 | 726.4 |
| **55** | {(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3-trifluormethoxy-benzylester | AV: 1Aa, b, d, g, 3A, 4 | 741 | 742.3 |
| **56** | ((S)-1 -[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2-trifluormethoxy-benzylester | AV: 1Aa, b, d, g, 3A, 4 | 741 1 | 742.3 |
| **58** | (R)-6,8-Dichlor-3-{(S)-2-[2-(4-fluor-phenyl)-ethylamino]-3-methyl-pentanoylamino}-2,3,4,9-tetrahydro-1H-carbazole-3-carbonsäure-((S)-1-carbamoyl-2-methyl-butyl)-amid | AV: 1Aa, b, d, h, 3A, 4 | 645 | 647.1 |
| **59** | (R)-6,8-Dichlor-3-{(S)-2-[2-(2,6-difluor-phenyl)-acetylamino]-3-methyl-pentanoylaminol-2,3,4,9-tetrahydro-1H-carbazole-3-carbonsäure-((S)-1-carbamoyl-2-methyl-butyl)-amid | AV: 1Aa, b, d, f, 3A, 4 | 677 | 678.5 |
| **61** | (R)-6,8-Dichlor-3-1(S)-2-[2-(3-fluor-phenyl)-ethylamino]-3-methyl-pentanoylamino}-2,3,4,9-tetrahydro-1H-carbazole-3-carbonsäure((S)-1-carbamoyl-2-methyl-butyl)-amid | AV: 1Aa, b, d, h, 3A, 4 | 645 | 646.3 |
| **63** | (R)-8-Chlor-6-fluor-3-{(S)-2-[2-(4-fluor-phenyl)-ethylamino]-3-methyl-pentanoylamino}-2,3,4,9-tetrahydro-1H-carbazole-3-carbonsäure-((S)-1-carbamoyl-2-methyl-butyl)-amid | AV: 1Aa, b, d, h, 3A, 4 | 629 | 630.4 |
| **64** | {(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyl)-carbaminsäure-4-fluor-benzylester | AV: 1Aa, b, d, g, 3A, 4 d, g, 3A, 4 | 659 | 660.3 |
| **69** | (R)-8-Chlor-6-fluor-3-{(S)-2-[2-(2-fluorphenyl)-acetylamino]-3-methyl-pentanoylamino}-2,3,4,9-tetrahydro-1H-carbazol-3-carbonsäure-((S)-1-carbamoyl-2-methyl-butyl)-amid | AV: 1Aa, b, d, f, 3A, 4 | 643 | 644.4 |
| **74** | (R)-3-{(S)-2-[2-(2,6-Difluorphenyl)-acetylamino]-3-methyl-pentanoylamino}-8-trifluormethyl-2,3,4,9-tetrahydro-1H-carbazol-3-carbonsäure-((S)-1-carbamoyl-2-methyl-butyl)-amid | AV: 1Aa, b, d, f, 3A, 4 | 677 | 678.2 |
| **75** | (R)-3-{(S)-2-[2-(2-Fluorphenyl)-acetylamino]-3-methyl-pentanoylamino}-8-trifluormethyl-2,3,4,9-tetrahydro-1H-carbazol-3-carbonsäure-((S)-1-carbamoyl-2-methyl-butyl)-amid | AV: 1Aa, b, d, f, 3A, 4 | 659 | 660.4 |

### II. Nachweis der LHRH-antagonistischen Wirkung erfindungsgemäßer Verbindungen

### II.1A LHRH-Rezeptor-Ligand-Bindungsassay (Membranpräparationen aus Rattenhypophysenzellen)

### Testung

Zur Durchführung von heterologen Kompetitionsexperimenten wurden Membranpräparationen aus Rattenhypophysenzellen verwendet, die den LHRH-Rezeptor natürlicherweise stark exprimieren. Als Ligand wurde hierbei [¹²⁵I][D-Trp⁶]-LHRH in einer Konzentration von 0,05 nM verwendet. Zur Kompetition wurde unmarkiertes [D-Trp⁶]-LHRH in einer Konzentration von 1µM bzw. die Testsubstanz in der gewünschten Konzentration verwendet. Nach einer Inkubationszeit von 90 Minuten bei 4°C erfolgte die Messung des gebundenen Liganden per Szintillation (Halmos et al., Proc. Natl. Acad. Sci. USA, 1996, 93, 2398).

### Auswertung

Als Ergebnis resultierte der prozentuale Anteil des gebundenen Liganden in Anwesenheit des Kompetitors im Verhältnis zum spezifisch gebundenen Anteil der Kontrolle (siehe Ergebnis für ausgewählte Verbindungen in **Tabelle 3**). Mittels nichtlinearer Regressionsanalyse der Kompetitionskurven wurden EC₅₀ Werte berechnet.

**Tabelle 3: LHRH Rezeptor-Ligand Bindungsassay Versuchsergebnisse, EC₅₀ und Ki Werte für ausgewählte Substanzen**

| Substanz | EC₅₀ (M) |
|---|---|
| **48** | 8.0 x 10⁻⁹ |
| **75** | 5.9 x 10⁻¹⁰ |

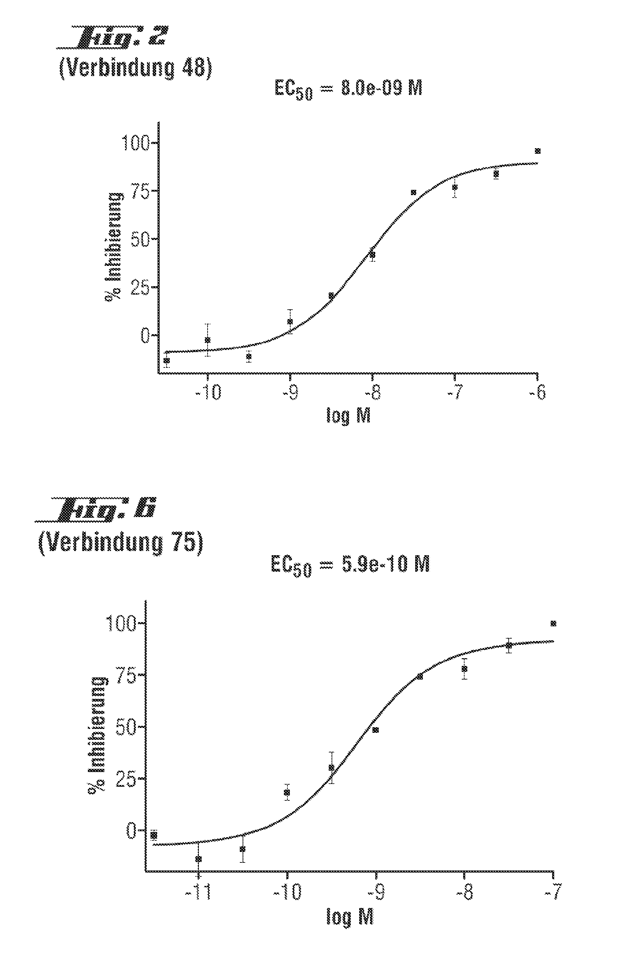

In den **Abbildungen 2** und **6** sind gemessene Kompetitionskurven LHRH-Rezeptor-Ligand Bindungsassay mit [¹²⁵I][D-Trp6]-LH-RH und den ausgewählten Substanzen (**48** und **75**) dargestellt.

### II.1B LHRH-Rezeptor-Ligand-Bindungsassay (transfizierte LTK-Zellen)

### Materialien:

¹²⁵I Triptorelin wurde von Biotrend (Köln, Deutschland) bezogen. Die spezifische Aktivität betrug jeweils 2.13 Ci/mmol
*Alle anderen Chemikalien werden aus kommerziellen Quellen im höchsten verfügbaren Reinheitsgrad bezogen.*

### Zellkultur:

LTK⁻ Zellen (Maus Fibroblasten: ATCC No. CCL-1.3), die mit dem Ratten LHRH-Rezeptor transfiziert wurden, werden in DMEM-Medium (Invitrogen Life Technologies, Deutschland) mit Penicillin (100 I.U./ml), Streptomycin (0,1 mg/ml) und Glutamin (0,01 mol/l) und 10% fötalem Kälberserum (FCS; Invitrogen Life Technologies, Deutschland) auf Plastikgewebekulturplatten (Nunc, Deutschland, 245 x 245 x 20 mm) kultiviert.

### Testung:

80% konfluente Zellkulturplatten werden zweimal mit 50ml Phosphat-gepufferter Salzlösung (PBS) gewaschen und nachfolgend mit 0.01 M EDTA Lösung abgelöst. Die Zellen werden durch Zentrifugation bei 200xg für 5 min in einer Laborzentrifuge (Kendro, Deutschland) pelletiert. Das Zellpellet wird in 3ml Bindungsmedium (DMEM; 10mM Hepes; 0.5% BSA; 0.1% NaN₃; 1g/l Bacitracin (frisch zugeben, Stock 100x); 0.1g/l SBTI (frisch zugeben, Stock 1000x)) resuspendiert und die Zellzahl mittels Trypanblaufärbung in einer Neubauer Zählkammer bestimmt. Die Zellsuspension wird mit Bindungsmedium auf eine Konzentration von 5x10⁵ Zellen / 0.05ml eingestellt.
Bindungsuntersuchungen für Kompetitionskurven werden als Duplikate durchgeführt. Die Testsubstanzen werden als 10mM DMSO Lösungen eingesetzt. Sie werden auf das 4 fache der eingesetzten Endkonzentration mit Bindungsmedium verdünnt. 25µl der Substanzverdünnnung werden mit 25µl Tracerlösung (¹²⁵I Triptorelin oder ¹²⁵I Cetrorelix) gemischt. Die Konzentration an Tracer ist auf ca. 50000cpm (gemessen im Cobra II, Gamma-Counter, PE Liefe Science, Deutschland) im Endreaktionsvolumen von 100µl eingestellt.
In 650µl Spitzbodenröhrchen (Roth, Deutschland) werden 200µl Silkon/Parafinöl Mischung (84%:16%) vorgelegt. Darauf werden 50µl der Zellsuspension pipettiert und nachfolgend 50µl der Testsubstanz / Tracer Mischung. Die Röhrchen werden verschlossen und 60min bei 37°C in einem Inkubator über Kopf drehend inkubiert. Nach Inkubation werden die Proben in einer Zentrifuge (Kendro, Deutschland) bei 900 Upm zentrifugiert und nachfolgend in flüssigem N₂ schockgefroren. Die Spitze mit dem Zellpellet wird abgeschnitten und in vorbereitete Zählröhrchen (Roth, Deutschland) überführt. Das restliche Spitzbodenröhrchen mit dem verbliebenen Überstand wird ebenfalls in ein Zählröhrchen überführt. Die Messung erfolgt im Y counter für 1min / Probe.
Die Auswertung der Proben erfolgt nach Berechnung der spezifischen Bindung im Vergleich zu unbehandelten Zellen, nach Abzug der unspezifischen Bindung (Überschuß unmarkierter Ligand, 1µM) mittels GrapPad Prism oder alternativ mittels OMMM Software.

**Tabelle 4: LHRH Rezeptor-Ligand Bindungsassay Versuchsergebnisse, EC₅₀ Werte für eine Reihe ausgewählter Beispielsubstanzen**

| **Beispiel-Nr.** | **Bindung an r-LHRHR EC₅₀ [µM]** | **Name gem. Autonom** |
|---|---|---|
| **1** | 0,2609 | {1-[3-(Carbamoyl-2-methyl-propylcarbamoyl)-6,8-dichlor-2,3,4,9-tetrahydro-1H-carbazol-3-yl-carbamoyl]-2-methyl-propyl}-carbaminsäure-4-chlor-benzylester |
| **2** | 0,2051 | {1-[3-(Carbamoyl-2-methyl-propylcarbamoyl)-6,8-dichlor-2,3,4,9-tetrahydro-1H-carbazol-3-yl-carbamoyl]-2-methyl-butyl}-carbaminsäure-4-chlor-benzylester |
| **3** | 0,0472 | {1-[3-(Carbamoyl-2-methyl-butylcarbamoyl)-6,8-dichlor-2,3,4,9-tetrahydro-1H-carbazol-3-yl-carbamoyl]-2-methyl-butyl}-carbaminsäure-4-chlor-benzylester |
| **4** | 0,0048 | 6,8-Dichlor-3-{2-[2-(2-fluorphenyl)-acetylamino]-3-methyl-pentanoylamino}-2,3,4,9-tetrahydro-1H-carbazol-3-carbonsäure-(1-carbamoyl-2-methyl-butyl)-amid |
| **5** | 0,09835 | 6,8-Dichlor-3-{2-[2-(3-fluorphenyl)-acetylamino]-3-methyl-pentanoylamino}-2,3,4,9-tetrahydro-1H-carbazol-3-carbonsäure-(1-carbamoyl-2-methyl-butyl)-amid |
| **6** | 0,1037 | {1-[3-(1-Carbamoyl-2-methyl-butylcarbamoyl)-6,8-dichlor-2,3,4,9-tetrahydro-1H-carbazol-3-yl-carbamoyl]-2-methyl-butyl}-carbaminsäure-2-chlor-benzylester |
| **16** | 0,62805 | 6,8-Dichlor-3-{2-[3-(4-fluorphenyl)-propionylamino]-3-methyl-pentanoylamino}-2,3,4,9-tetrahydro-1H-carbazol-3-carbonsäure-(1-carbamoyl-2-methyl-propyl)-amid |
| **17** | 0,5252 | 5-[3-(1-Carbamoyl-2-methyl-propylcarbamoyl)-6,8-dichlor-2,3,4,9-tetrahydro-1H-carbazol-3-yl-carbamoyl]-5-[3-(4-fluorphenyl)-propionylamino]-pentyl-ammonium trifluoracetat |
| **22** | 0,02465 | 6,8-Dichlor-3-[2-[3-(2-fluor-phenyl)-propionylamino]-4-(4-hydroxy-phenyl)-butyrylamino]-2,3,4,9-tetrahydro-1H-carbazol-3-carbonsäure-(1-carbamoyl-2-methyl-butyl)-amid |
| **23** | 0,0729 | 6,8-Dichlor-3-{2-[3-(2-fluor-phenyl)-propionylamino]-3-methyl-pentanoylaminol-2,3,4,9-tetrahydro-1H-carbazol-3-carbonsäure-(1-carbamoyl-2-methyl-butyl)-amid |

### 11.2 Inhibition der LHRH-induzierten LH-Sekretion aus Ratten-Hypophysen-Zellen in vitro

### Materialien

Zur Messung der LH-Konzentration in Zellkulturüberständen wurde der "Rat luteinising hormone (rLH) enzymeimmunoassay (EIA) system" ELISA (RPN 2562) der Firma Amersham Pharmacia Biotech verwendet. Alle anderen Chemikalien wurden aus kommerziellen Quellen im höchsten verfügbaren Reinheitsgrad verwendet.

### Zellkultur

Juvenile männliche Wistar Ratten (Harlan Winkelmann, Germany) wurden durch Dekapitieren getötet, die Hypophysen entnommen und in Hanks Puffer (HBSS) mit 0.3% BSA und 10 mM HEPES (pH 7,4) aufgenommen. Zur Durchführung eines Experimentes wurden Hypophysen von 20 Ratten benötigt. Zur Trennung der Zellen des Hypophysenvorderlappens vom restlichen Gewebe erfolgte eine 30-minütige Inkubation bei 37°C in Hanks Puffer mit 10mM HEPES (pH 7.4), 0.3% BSA, 1mg/ml Hyaluronidase (Typ VIII), 1mg/ml Trypsin Inhibitor aus Sojabohnen, 10µg/ml DNAsel und 1mg/ml Papain. Mittels steriler Pasteurpipette wurden die Zellen dispergiert und anschließend durch Zentrifugation pelletiert. Die Aussaat der Zellen erfolgte in einer Dichte von 2.5 x10⁵ Zellen /Well einer mit Kollagen beschichteten 48-Well-Platte (Becton Dickinson) in DMEM Medium (Invitrogen Life Technologies, Deutschland) mit 10% fötalem Kälberserum (FCS; Invitrogen Life Technologies, Deutschland), 10ml/l nicht-essentiellen Aminosäuren und 10ml/l Pen/Strep (Penicillin/Streptomycin).

### Testung

Nach einer Inkubation für 48h bei 37°C, 5% CO₂ und 95% Luftfeuchtigkeit erfolgte ein Mediumwechsel. Das Medium wurde ersetzt durch LHRH-haltiges (10nM) Medium bzw. Medium mit LHRH (10nM) und Testsubstanz in der angegebenen (Tabelle) Konzentration. Nach einer weiteren 3-stündigen Inkubation wurde der Zellüberstand gesammelt und bei -20°C weggefroren. Die Bestimmung des LH Gehaltes erfolgte mittels ELISA in Dreifachbestimmung laut Angaben des Herstellers (Amersham Pharmacia Biotech).
"% Inhibition" in der nachfolgenden Tabelle beschreibt den Quotienten aus der LHRH-stimulierten LH-Sekretion mit ("LH (ng/ml)") bzw. ohne Zusatz der Testsubstanz. Die Werte stammen aus unterschiedlichen, unabhängigen Experimenten.

**Tabelle 5: Inhibition der LHRH-stimulierten LH-Sekretion aus RattenHypophysenzellen durch ausgewählte Substanzen**

| Substanz | Konzentration | LH (ng/ml) ± SD | % Inhibition |
|---|---|---|---|
| **4** | 3.3 x 10⁻⁸ M | 79.3 ± 5.7 | 67 % |
| **4** | 3.3 x 10⁻⁹ M | -- | -- |

## Patentansprüche

1. Neue Tetrahydrocarbazol-Verbindungen der allgemeinen Formel (I): 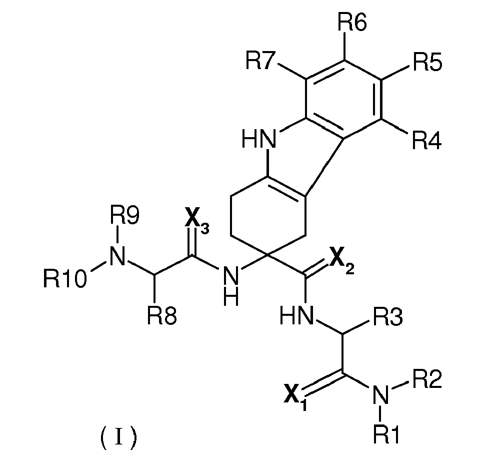 worin:
X₁ O bedeutet,
X₂ und X₃ unabhängig voneinander O oder geminal verknüpftes H₂ bedeuten,
R1 und R2 unabhängig voneinander ausgewählt sind aus der Gruppe bestehend aus -H, Aryl-, Alkyl- und Arylalkyl-Resten, welche gegebenenfalls in der Alkyl- und/oder Aryl-Gruppe mit bis zu 3 Substituenten, unabhängig ausgewählt aus der Gruppe bestehend aus -Hal, -CN, und -O-alkyl, substituiert sind, wobei R1 und R2 insbesondere jeweils ein Wasserstoffatom bedeuten,
R3 und R8 einen Alkyl-, Arylalkyl- oder Heteroarylalkyl-Rest bedeutet, welche gegebenenfalls mit bis zu 3 Substituenten, unabhängig ausgewählt aus der Gruppe bestehend aus -Hal, -CN, -CO-O-R12, -CO-NR12R12', -OH, -OR13, -O-CO-R13, -O-SO₂-OR12, -O-SO₂-R12, -SO₂-OR12, -SO-R12, -O-PO(OR12)(OR12'), -O-PO(NR12R12')₂, -O-CO-O-R13, -O-CO-NR12R12', -O-CS-NR12R12', -S-R12, -NR12R12', -NH-CO-R13, -NH-SO₂-R12, -NH-CO-O-R13, -NH-CO-NHR12, -NH-C(NH)-NH₂ substituiert sind,
R4, R5, R6 und R7 unabhängig voneinander ausgewählt sind aus der Gruppe bestehend aus H, -Hal, -CN, -CONH₂, -COOH, -CF₃, -O-Alkyl, -OCF₃, -NO₂, und Alkyl-, Arylalkyl-, und Heteroarylalkyl-Resten;
R9 ein Wasserstoffatom, einen Alkyl-, einen Aryl-, einen Heteroaryl-, einen Arylalkyl- oder einen Heteroarylalkyl-Rest, vorzugsweise ein Wasserstoffatom, bedeutet;
R10 den Rest -R11, -CO-R11, -CO-OR11, -CO-NHR11, -C(NH)-NHR11, -SO₂-R11, oder -SO₂-NHR11 bedeutet;
R11 ein Alkyl-, ein Aryl-, ein Heteroaryl-, ein Arylalkyl- oder ein Heteroarylalkyl-Rest bedeutet, welche gegebenenfalls mit einem oder mehreren Substituenten, unabhängig ausgewählt aus der Gruppe bestehend aus -Hal, -CN, -Alkyl, -CF₃, -OCF₃, -OH, -O-Alkyl, und -O-(CH₂CH₂-O)ₙ-CH₃, substituiert sind, wobei der Arylalkyl- oder Heteroarylalkyl-Rest in der Aryl- bzw. Heteroaryl-Gruppe mit einem oder mehreren Substituenten, unabhängig ausgewählt aus der Gruppe bestehend aus -Hal, -CN, -Alkyl, -CF₃, -OCF₃, -OH, -O-Alkyl, und -O-(CH₂CH₂-O)ₙ-CH₃ substituiert ist;
R12 und R12' unabhängig voneinander -H, oder einen Alkyl-, Arylalkyl-, Aryl-, Heteroarylalkyl-, oder Heteroaryl-Rest bedeuten, und vorzugsweise H bedeuten,
R13 ausgewählt ist aus einem Alkyl-, Arylalkyl-, Aryl-, Heteroarylalkyl-, und Heteroaryl-Rest, oder die Gruppe -(CH₂CH₂-O)ₙ-CH₃ bedeutet; und
n für eine ganze Zahl von 1 bis 10, vorzugsweise 1 bis 6, steht;
sowie physiologisch verträgliche Salze, Derivate oder Analoga der Verbindung der allgemeinen Formeln (I), wobei die Salze erhältlich sind durch Neutralisation der Basen mit anorganischen oder organischen Säuren bzw. durch Neutralisation der Säuren mit anorganischen oder organischen Basen,
wobei die Verbindung der allgemeinen Formel (I) sowie ihre Salze, Derivate oder Analoga in Form ihrer Racemate, in Form der reinen Enantiomeren und/oder Diasteromeren oder in Form von Mischungen dieser Enantiomeren und/oder Diasteromeren, in Form der Tautomeren, deren Solvate und Hydrate und deren polymorphen Formen vorliegen können.

2. Verbindungen der allgemeinen Formel (I) gemäß Anspruch 1, wobei die Verbindungen der allgemeinen Formel (I) an dem durch -NH-CX3- und -CX2-NH- substituierten Kohlenstoffatom in R-Konfiguration vorliegen.

3. Verbindungen der allgemeinen Formel (I) gemäß Anspruch 2, wobei die Verbindungen der allgemeinen Formel (I) an dem durch -CX₃-NH-, -R8 und -NR9R10 substituierten Kohlenstoffatom in S-Konfiguration und an dem durch -NH-CX₂-, -R3 und -CX₁-NR1R2 substituierten Kohlenstoffatom ebenfalls in S-Konfiguration vorliegen.

4. Verbindungen der allgemeinen Formel (I) gemäß einem der Ansprüche 1 bis 4, wobei
R10 den Rest -R11, -CO-R11, -CO-OR11, -CO-NHR11, -C(NH)-NHR11, -SO₂-R11, oder -SO₂NHR11 bedeutet,
R11 ein Arylalkyl- oder Heteroarylalkyl-Rest ist, der in der Aryl- bzw. Heteroaryl-Gruppe mit einem oder mehreren Substituenten, unabhängig ausgewählt aus der Gruppe bestehend aus Hal, -CN, -Alkyl, -CF₃, -OCF₃, -OH, -O-Alkyl, und -O-(CH₂CH₂-O)ₙ-CH₃ substituiert ist, und
R8 einen Alkyl-, Arylalkyl- oder Heteroarylalkyl-Rest bedeutet, der gegebenenfalls mit bis zu 3 Substituenten, unabhängig ausgewählt aus der Gruppe bestehend aus-Hal, -CN, -CO-O-R12, -CO-NR12R12', -OH, -O-R13, -O-CO-R13, -O-SO₂-OR12, -SO₂-OR12, -O-SO₂-R12, -SO-R12, -O-PO(OR12)(OR12'), -O-PO(NR12R12')₂, -O-CO-O-R13, -O-CO-NR12R12', -O-CS-NR12R12', -S-R12, -N R12R12', -NH-CO-R13, -NH-SO₂-R12, -NH-CO-O-R13, -NH-CO-NHR12, -NH-C(NH)-NH₂ substituiert ist.

5. Verbindungen der allgemeinen Formel (1) gemäß einem der Ansprüche 1 bis 4, wobei mindestens einer, vorzugsweise mindestens zwei der Reste R4, R5, R6, und R7, vorzugsweise R5 und R7, keine Wasserstoffatome sind.

6. Verbindungen der allgemeinen Formel (I) gemäß Anspruch 5, wobei die Reste R4 und R6 jeweils ein Wasserstoffatom bedeuten und die Reste R5 und R7 unabhängig voneinander ausgewählt sind aus der Gruppe bestehend aus -H, -Hal, -CN, -CF₃, -O-Alkyl und -OCF₃, und vorzugsweise -H, -Hal oder -CF₃ bedeuten.

7. Verbindungen der allgemeinen Formel (I) gemäß Anspruch 6, wobei der Rest R5 H oder Hal und der Rest R7 Hal oder -CF₃ bedeutet.

8. Verbindungen der allgemeinen Formel (I) gemäß einem der Ansprüche 1 bis 7, wobei X₂ und X₃ jeweils O bedeuten.

9. Verbindungen der allgemeinen Formel (I) gemäß einem der Ansprüche 1 bis 8, wobei R3 einen C₁-C₆-Alkyl-Rest, vorzugsweise einen C₁-C₄-Alkyl-Rest bedeutet.

10. Verbindungen der allgemeinen Formel (I) gemäß einem der Ansprüche 1 bis 9, wobei R1 und R2 jeweils ein Wasserstoffatom bedeuten.

11. Verbindungen der allgemeinen Formel (I) gemäß einem der Ansprüche 1 bis 10, wobei R9 und, sofern vorhanden, R12 und R12' jeweils ein Wasserstoffatom bedeuten.

12. Verbindungen der allgemeinen Formel (I) gemäß einem der Ansprüche 1 bis 11, wobei R13 einen Phenyl-C₁-C₄-alkyl-Rest, oder die Gruppe -(CH₂CH₂-O)ₙ-CH₃ bedeutet.

13. Verbindungen der allgemeinen Formel (I) gemäß einem der Ansprüche 1 bis 12, wobei R10 den Rest -CO-R11 oder -CO-OR11 oder den Rest R11 bedeutet.

14. Verbindungen der allgemeinen Formel (I) gemäß einem der Ansprüche 1 bis 13, wobei R11 einen Phenyl-C₁-C₄-alkyl-Rest, vorzugsweise einen Benzyl- oder Phenylethyl-Rest bedeutet, der in der Phenyl-Gruppe gegebenenfalls mit einem bis drei, vorzugsweise einem oder zwei, Substituenten, unabhängig ausgewählt aus der Gruppe bestehend aus -Hal, -C1-C4-Alkyl, -CF3, -OCF3, -OH, -O-C1-C4-Alkyl und -O-(CH2CH2-O)n-CH3 substituiert ist.

15. Verbindungen der allgemeinen Formel (I) gemäß einem der Ansprüch 1 bis 3, wobei
X₁ O bedeutet,
X₂ und X₃ jeweils O bedeuten,
R1 und R2 jeweils ein Wasserstoffatom bedeuten,
R3 einen C₁-C₆-Alkyl-Rest, vorzugsweise C₁-C₄-Alkyl-Rest bedeutet,
R4 und R6 jeweils ein Wasserstoffatom bedeuten,
R5 entweder ein Wasserstoffatom oder Hal bedeutet,
R7 entweder Hal oder -CF₃ bedeutet,
R9 ein Wasserstoffatom bedeutet,
R10 den Rest -CO-R11 oder-CO-OR11 oder den Rest R11 bedeutet,
R11 ein Phenyl-C₁-C₄-alkyl-Rest, vorzugsweise ein Benzyl- oder Phenylethyl-Rest ist, der in der Phenyl-Gruppe gegebenenfalls mit einem bis drei, vorzugsweise einem oder zwei Substituenten, unabhängig ausgewählt aus der Gruppe bestehend aus -Hal, -C₁-C₄-Alkyl, -CF₃, -OCF₃, -OH, -O-C₁-C₄-Alkyl und -O-(CH₂CH₂-O)ₙ-CH₃ substituiert ist, und
R8 einen C₁-C₆-Alkyl-, vorzugsweise einen C₁-C₄-Alkyl-Rest bedeutet; und
n für eine ganze Zahl von 1 bis 6, vorzugsweise 4, steht.

16. Verbindungen der allgemeinen Formel (I) gemäß Anspruch 1 oder 15, ausgewählt aus der Gruppe bestehend aus:
{(S)-1-[(R)-3-((S)-1-Carbamoyl-2-methyl-propylcarbamoyl)-6,8-dichlor-2,3,4,9-tetrahydro-1H-carbazol-3-yl-carbamoyl]-2-methyl-propyl}-carbaminsäure-4-chlor-benzylester (**1**),
{(S)-1-[(R)-3-((S)-1-Carbamoyl-2-methyl-propylcarbamoyl)-6,8-dichlor-2,3,4,9-tetrahydro-1H-carbazol-3-yl-carbamoyl]-2-methyl-butyl}-carbaminsäure-4-chlor-benzylester (**2**),
{(S)-1-[(R)-3-((S)-Carbamoyl-2-methyl-butylcarbamoyl)-6,8-dichlor-2,3,4,9-tetrahydro-1H-carbazo1-3-yl-carbamoyl]-2-methyl-butyl}-carbaminsäure-4-chlor-benzylester (**3**),
(R)-6,8-Dichlor-3-1(S)-2-[2-(2-fluorphenyl)-acetylamino]-3-methyl-pentanoylaminol-2,3,4,9-tetrahydro-1H-carbazol-3-carbonsäure-((S)-1-carbamoyl-2-methyl-butyl)-amid (**4**),
(R)-6,8-Dichlor-3-1(S)-2-[2-(3-fluorphenyl)-acetylamino]-3-methyl-pentanoylaminol-2,3,4,9-tetrahydro-1H-carbazol-3-carbonsäure-((S)-1-carbamoyl-2-methyl-butyl)-amid (**5**),
{(S)-1-[(R)-3-((S)-Carbamoyl-2-methyl-butylcarbamoyl)-6,8-dichlor-2,3,4,9-tetrahydro-1H-carbazol-3-yl-carbamoyl]-2-methyl-butyl}-carbaminsäure-2-chlor-benzylester (**6**),
(R)-6,8-Dichlor-3-1(S)-2-[3-(4-fluorphenyl)-propionylamino]-3-methyl-pentanoylaminol-2,3,4,9-tetrahydro-1H-carbazol-3-carbonsäure-((S)-1-carbamoyl-2-methyl-propyl)-amid (**16**),
(S)-5-[(R)-3-((S)-1-Carbamoyl-2-methyl-propylcarbamoyl)-6,8-dichlor-2,3,4,9-tetrahydro-1H-carbazol-3-yl-carbamoyl]-5-[3-(4-fluorphenyl)-propionylamino]-pentyl-ammonium trifluoracetat **(17),**
(S)-6,8-Dichlor-3-1(S)-2-[3-(2-hydroxy-phenyl)-propionylamino]-3-methyl-pentanoylaminol-2,3,4,9-tetrahydro-1H-carbazol-3-carbonsäure-((S)-1-carbamoyl-2-methyl-butyl)-amid **(18),**
(R)-6,8-Dichlor-3-((S)-2-{3-[2-(2-{2-[2-(2-methoxy-ethoxy)-ethoxy]-ethoxy}-ethoxy)-phenyl]-propionylamino}-3-methyl-pentanoylamino)-2,3,4,9-tetrahydro-1H-carbazol-3-carbonsäure-((S)-1-carbamoyl-2-methyl-butyl)-amid (**20**),
(R)-6,8-Dichlor-3-((S)-2-{2-[2-(2-{2-[2-(2-methoxy-ethoxy)-ethoxy]-ethoxy}-ethoxy)-phenyl]-acetylamino}-3-methyl-pentanoylamino)-2,3,4,9-tetrahydro-1H-carbazol-3-carbonsäure-((S)-1-carbamoyl-2-methyl-butyl)-amid (**21**),
(R)-6,8-Dichlor-3-[(S)-2-[3-(2-fluor-phenyl)-propionylamino]-4-(4-hydroxy-phenyl)-butyrylamino]-2,3,4,9-tetrahydro-1H-carbazol-3-carbonsäure-((S)-1-carbamoyl-2-methyl-butyl)-amid (**22**),
(R)-6,8-Dichlor-3-{(S)-2-[3-(2-fluor-phenyl)-propionylamino]-3-methyl-pentanoylaminol-2,3,4,9-tetrahydro-1H-carbazol-3-carbonsäure-((S)-1-carbamoyl-2-methyl-butyl)-amid (**23**),
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3-methyl-benzylester (**26**),
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2,6-difluor-benzylester (**27**),
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3,5-difluor-benzylester (**28**),
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3,5-dichlor-benzylester (**29**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-3-fluor-benzylester (**30**),
{(S)-1-[(R)-3-((S)-1-Carbamoyl-2-methyl-butylcarbamoyl)-6 ,8-dichlor-2,3,4,9-tetrahydro-1H-carbazol-3-ylcarbamoyl]-2-methyl-butyl}-carbaminsäure-2-fluor-benzylester (**31**),
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-3-chlor-benzylester (**32**),
{(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyl}-carbaminsäure-3,5-difluor-benzylester (**33**),
{(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyll-carbaminsäure-3-fluor-benzylester (**34**),
{(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyl}-carbaminsäure-2-fluor-benzylester (**35**),
[(S)-1-[(R)-3-((S)-1-Carbamoyl-2-methyl-butylcarbamoyl)-6,8-dichlor-2,3,4,9-tetrahydro-1H-carbazol-3-ylcarbamoyl]-3-(4-hydroxy-phenyl)-propyl]-carbaminsäure-3-fluor-benzylester (**37**),
[(S)-1-[(R)-3-((S)-1-Carbamoyl-2-methyl-butylcarbamoyl)-6,8-dichlor-2,3,4,9-tetrahydro-1H-carbazol-3-ylcarbamoyl]-3-(4-hydroxyphenyl)-propyl]-carbaminsäure-2-fluor-benzylester (**38**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-2-(2-fluor-phenyl)-ethylester (**40**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-3-methoxy-benzylester (**47**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-4-fluor-benzylester (**48**),
{(S)-1-[(R)-3-((S)-1-Carbamoyl-2-methyl-butylcarbamoyl)-6,8-dichlor-2,3,4,9-tetrahydro-1H-carbazol-3-ylcarbamoyl]-2-methyl-butyl}-carbaminsäure-2-methyl-benzylester **(49),**
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-2,3-dimethoxy-benzylester **(50),**
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-2-methoxybenzylester **(51),**
(R)-6,8-Dichlor-3-1(S)-2-[2-(2-fluor-phenyl)-ethylamino]-3-methyl-pentanoylaminol-2,3,4,9-tetrahydro-1H-carbazole-3-carbonsäure-((S)-1-carbamoyl-2-methyl-butyl)-amid **(52),**
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-2-trifluormethyl-benzylester (**53**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-3-trifluormethyl-benzylester (**54**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-3-trifluormethoxy-benzylester (**55**),
{(S)-1-[(R)-3-((S)-1-Carbamoyl-2-methyl-butylcarbamoyl)-6,8-dichlor-2,3,4,9-tetrahydro-1H-carbazo1-3-ylcarbamoyl]-2-methyl-butyl}-carbaminsäure-2-trifluormethoxy-benzylester (**56**),
(R)-6,8-Dichlor-3-1(S)-2-[2-(4-fluor-phenyl)-ethylamino]-3-methyl-pentanoylaminol-2,3,4,9-tetrahydro-1H-carbazole-3-carbonsäure-((S)-1-carbamoyl-2-methyl-butyl)-amid (**58**),
(R)-6,8-Dichlor-3-1(S)-2-[2-(2,6-difluor-phenyl)-acetylamino]-3-methyl-pentanoylaminol-2,3,4,9-tetrahydro-1H-carbazole-3-carbonsäure-((S)-1-carbamoyl-2-methyl-butyl)-amid (**59**),
(R)-6,8-Dichlor-3-{(S)-2-[2-(3-fluor-phenyl)-ethylamino]-3-methyl-pentanoylaminol-2,3,4,9-tetrahydro-1H-carbazole-3-carbonsäure((S)-1-carbamoyl-2-methyl-butyl)-amid (**61**),
(R)-8-Chlor-6-fluor-3-{(S)-2-[2-(4-fluor-phenyl)-ethylamino]-3-methyl-pentanoylamino}-2,3,4,9-tetrahydro-1H-carbazole-3-carbonsäure-((S)-1-carbamoyl-2-methyl-butyl)-amid (**63**),
{(S)-1-[(R)-3-((S)-1-Carbamoyl-2-methyl-butylcarbamoyl)-8-chlor-6-fluor-2,3,4,9-tetrahydro-1H-carbazol-3-ylcarbamoyl]-2-methyl-butyll-carbaminsäure-4-fluor-benzylester (**64**),
(R)-8-Chlor-6-fluor-3-{(S)-2-[2-(2-fluorphenyl)-acetylamino]-3-methyl-pentanoylamino}-2,3,4,9-tetrahydro-1H-carbazol-3-carbonsäure-((S)-1-carbamoyl-2-methyl-butyl)-amid (**69**),
(R)-3-{(S)-2-[2-(2,6-Difiuorphenyl)-acetyiamino]-3-methyl-pentanoyiamino}-8-trifluormethyl-2,3,4,9-tetrahydro-1H-carbazol-3-carbonsäure-((S)-1-carbamoyl-2-methyl-butyl)-amid (**74**),
(R)-3-{(S)-2-[2-(2-Fiuorphenyl)-acetylamino]-3-methyl-pentanoylamino}-8-trifiuormethyl-2,3,4,9-tetrahydro-1H-carbazol-3-carbonsäure-((S)-1-carbamoyl-2-methyl-butyl)-amid (**75**).

17. Pharmazeutische Zusammensetzung, die eine pharmakologisch aktive Menge mindestens einer Verbindung gemäß einem der Ansprüche 1 bis 16 umfasst.

18. Pharmazeutische Zusammensetzung gemäß Anspruch 17, wobei der Wirkstoff in einer Einheitsdosis von 0,001 mg bis 100 mg pro kg Körpergewicht eines Patienten vorliegt.

19. Pharmazeutische Zusammensetzung gemäß Anspruch 17 oder 18, wobei die Zusammensetzung weiterhin mindestens einen pharmazeutisch verträglichen Trägerstoff enthält.

20. Pharmazeutische Zusammensetzung gemäß einem der Ansprüche 17 bis 19, wobei die Zusammensetzung mindestens einen weiteren pharmakologisch aktiven Wirkstoff enthält.

21. Verbindungen gemäß einem der Ansprüche 1 bis 16 zur Verwendung als Arzneimittel.

22. Verwendung einer Verbindung gemäß einem der Ansprüche 1 bis 16 zur Herstellung eines Arzneimittels zur Behandlung oder Prophylaxe von durch einen G-Protein gekoppelten Rezeptor vermittelten Krankheitszuständen, oder von Krankheitszuständen, die durch Modulation dieses Rezeptors behandelt werden können.

23. Verwendung gemäß Anspruch 22, wobei es sich bei dem G-Protein gekoppelten Rezeptor um den LHRH Rezeptor handelt.

24. Verwendung gemäß Anspruch 22, wobei es sich bei den G-Protein gekoppelten Rezeptor um einen Rezeptor der Neurokinin Familie, insbesondere den NK₁ und/oder NK₂ Rezeptor handelt.

25. Verwendung gemäß Anspruch 23, wobei die Verbindung gemäß einem der Ansprüche 1 bis 16 als Antagonist des LHRH Rezeptors wirkt.

26. Verwendung gemäß Anspruch 24, wobei die Verbindung gemäß einem der Ansprüche 1 bis 16 als Antagonist des NK₁ und/oder des NK₂ Rezeptors wirkt.

27. Verwendung gemäß Anspruch 23 oder 25 zur Behandlung gutartiger oder bösartiger Tumorerkrankungen, in der männlichen Fertilitätskontrolle, in der Hormontherapie, in der Hormonersatztherapie, zur Behandlung und/oder Kontrolle von weiblicher Sub- oder Infertilität, zur kontrollierten ovariellen Stimulation bei einer In Vitro Fertilization, zur weiblichen Empfängnisverhütung sowie zum Schutz vor Nebenwirkungen durch die Chemotherapie.

28. Verwendung gemäß Anspruch 23 oder 25, wobei die durch den LHRH Rezeptor vermittelten Krankheitszustände oder die durch Modulation des LHRH Rezeptors behandelbaren Krankheitszustände ausgewählt sind aus der Gruppe umfassend: Benigne Prostata-Hyperplasie (BPH), Endometriose, Uterine Fibroide, Uterus-Myome, Endometrium Hyperplasie, Dysmenorrhoe, Dysfunktionelle Uterusblutungen (Menorrhagie, Metrorrhagie), Pubertas praecox, Hirsutismus, Polyzystisches Ovarielles Syndrom, hormonabhängige Tumorerkrankungen, HIV Infektionen bzw. AIDS, neurologischen oder neurodegenerativen Erkrankungen, ARC (AIDS related complex), Kaposi-Sarkom, Tumoren ausgehend vom Hirn und/oder Nervensystem und/oder Hirnhäuten, Demenz und der Alzheimer Erkrankung.

29. Verwendung gemäß Anspruch 28, wobei die hormonabhängigen Tumorerkrankungen ausgewählt sind aus der Gruppe umfassend: Prostatakrebs, Brustkrebs, Gebärmutterkrebs, Endometriumkrebs, Gebärmutterhalskrebs, Eierstockkrebs.

30. Verwendung gemäß Anspruch 24 oder 26 zur Behandlung und Vorbeugung von Brechreiz und Erbrechen, zur Behandlung von Schmerzen, Entzündungen sowie rheumatischen und arthritischen Krankheitsbildern.
